# EUROPEAN PATENT SPECIFICATION

(11) **EP 2 674 755 B1**
(45) Date of publication and mention of the grant of the patent: **26.07.2017**
(21) Application number: 12744724.1
(22) Date of filing: 10.02.2012
(51) Int. Cl.: G01N 33/68

(54) **METHOD FOR DIAGNOSING ACUTE LUNG INJURY**
VERFAHREN ZUR DIAGNOSE EINER AKUTEN LUNGENVERLETZUNG
PROCÉDÉ DE DIAGNOSTIC D'UNE LÉSION PULMONAIRE AIGUË

(30) Priority: 10.02.2011 JP 2011027679
(43) Date of publication of application: 18.12.2013
(73) Proprietor: Nagasaki University, Nagasaki-shi Nagasaki , 852-8521 (JP); Sapporo Medical University, Sapporo-shi, Hokkaido 060-0061 (JP)
(72) Inventor: KAKUGAWA, Tomoyuki, Nagasaki-shi, Nagasaki 852-8521 (JP); YOKOTA, Shin-ichi, Sapporo-shi, Hokkaido 060-8556 (JP)
(74) Representative: J A Kemp
(86) International application number: PCT/JP2012/053174
(87) International publication number: WO 2012/108538

(56) References cited:
- JP-A- 8 240 593
- JP-A- 8 240 593
- JP-A- 2008 122 276
- S YOKOTA: "Prevalence of HSP47 antigen and autoantibodies to HSP47 in the sera of patients with mixed connective tissue disease", BIOCHEMICAL AND BIOPHYSICAL RESEARCH COMMUNICATIONS, vol. 303, no. 2, 4 April 2003 (2003-04-04) , pages 413-418, XP055003908, ISSN: 0006-291X, DOI: 10.1016/S0006-291X(03)00352-8
- RICHARD D. FREMONT ET AL: "Acute Lung Injury in Patients With Traumatic Injuries: Utility of a Panel of Biomarkers for Diagnosis and Pathogenesis", THE JOURNAL OF TRAUMA: INJURY, INFECTION, AND CRITICAL CARE, vol. 68, no. 5, 1 May 2010 (2010-05-01), pages 1121-1127, XP055148745, ISSN: 0022-5282, DOI: 10.1097/TA.0b013e3181c40728
- HIDEO IWASAKA: "Three-step research strategies for ARDS: new target molecules-ACE2, HMGB1, and HSP47", JOURNAL OF ANESTHESIA, vol. 21, no. 1, 30 January 2007 (2007-01-30), pages 122-123, XP055120241, ISSN: 0913-8668, DOI: 10.1007/s00540-006-0475-9
- EUN JOO LEE ET AL: "The expression of HSPs, anti-oxidants, and cytokines in plasma and bronchoalveolar lavage fluid of patients with acute respiratory distress syndrome", CLINICAL BIOCHEMISTRY, ELSEVIER INC, US, CA, vol. 45, no. 6, 21 January 2012 (2012-01-21), pages 493-498, XP028471935, ISSN: 0009-9120, DOI: 10.1016/J.CLINBIOCHEM.2012.01.020 [retrieved on 2012-01-31]
- TOMOYUKI KAKUGAWA ET AL: "Serum heat shock protein 47 levels are elevated in acute interstitial pneumonia", BMC PULMONARY MEDICINE, BIOMED CENTRAL, LONDON, GB, vol. 14, no. 1, 21 March 2014 (2014-03-21) , page 48, XP021180712, ISSN: 1471-2466, DOI: 10.1186/1471-2466-14-48
- HIDEO IWASAKA: 'Dai 4 Kai Hai Surfactant Bunshi Byotai Kenkyukai - Hai Byohen no Shufuku Saisei eno Approach - Shinki Hai Shogai Inshi HMGB1 to Hai Sen'ika Inshi HSP47 kara Mita ARDS no Chiryo-ho no Kaihatsu' RESPIRATORY MOLECULAR MEDICINE vol. 10, no. 3, 2006, pages 204 - 208, XP008168908
- HIROSHI MIYAGAWA ET AL.: 'Dai 4 Sho ARDS no Kiso Shikkan Haiketsusho ni Tomonau ARDS' JOURNAL OF CLINICAL AND EXPERIMENTAL MEDICINE 2004, pages 121 - 125, 214, XP008170288
- HIDEO IWASAKA: 'What's New in SURGERY FRONTIER Dai 67 Kai Ensho no Bunshi Kiko to Netsu Shock Tanpakushitsu 4) Heiketsusho to Netsu Shock Tanpakushitsu' SURG FRONT vol. 17, no. 4, 2010, pages 380 - 383, XP008170434
- IWASAKA HIDEO: 'Three-step research strategies for ARDS: new target molecules-ACE2, HMGB1, and HSP47' J ANESTH vol. 21, no. 1, 2007, pages 122 - 123, XP055120241
- HIDEO IWASAKA: 'HMGB1 to Hai Shogai (ALI/ARDS)' SURGICAL TRAUMA & IMMUNOLOGICAL RESPONSES vol. 17, no. 2, 2008, pages 62 - 64, XP008170435
- YOKOTA S ET AL.: 'Prevalence of HSP47 antigen and autoantibodies to HSP47 in the sera of patients with mixed connective tissue disease' BIOCHEM BIOPHYS RES COMMUN vol. 303, no. 2, 2003, pages 413 - 418, XP055003908

## Description

### Technical Field

The present invention relates to an examination method for the diagnosis of acute lung injury, an acute lung injury diagnostic reagent and the like.

### Background Art

Acute lung injury (also referred to as ALI) is an inflammatory disease of the lung, which is caused by a disease that directly or indirectly injures the lung such as sepsis, pneumonia and the like, an injury such as trauma and the like, and/or aspiration and the like. While it shows non-specific symptoms, the main symptom is respiratory distress sometimes accompanied by cough or chest pain. Patients with acute lung injury typically show the above-mentioned symptoms in 24 - 48 hr from the initial injury or disease. In a severe case, it is deadly. The death rate is extremely high and is 30 - 40%, which becomes higher due to aging or complication.

Acute lung injury is basically diagnosed by clinical diagnosis. Since clinical findings of acute lung injury is similar to that of acute heart failure and lung infection, to confirm the diagnosis and specify the cause, it is considered that patients should further take ABG (artery blood gas test: measurement of oxygen partial pressure·CO₂ partial pressure, pH and the like of the blood) and chest radiography. However, acute lung injury is a disease that progresses rapidly and shows extremely poor prognosis, for which no effective treatment method has been established. In addition, complication with pneumothorax, severe bacterial infection of lung and the like may be developed. Therefore, an early diagnosis is particularly necessary.

Recently, KL-6 (syalilated sugar chain antigen), SP-D (Surfactant protein-D) and SP-A (Surfactant protein-A) are utilized as serum markers for more convenient and rapid diagnoses of acute lung injury. However, these are also markers of known chronic progressive interstitial pneumonia such as cryptogenic organizing pneumonia (COP), idiopathic usual interstitial pneumonia (idiopathic UIP), idiopathic nonspecific interstitial pneumonia (idiopathic NSIP), collagen vascular disease (CVD)-associated usual interstitial pneumonia (collagen vascular disease (CVD)-associated UIP), collagen vascular disease (CVD)-associated non-specific interstitial pneumonia (collagen vascular disease (CVD)-associated NSIP) and the like. There is no known marker having high specificity, which is capable of diagnosing acute lung injury by clearly distinguishing the disease from them.

Heat shock protein 47 (HSP47) is a molecular chaperone having specificity to collagen, and is known as a heat shock-induced stress protein present in the endoplasmic reticulum. Since expression of HSP47 is coupled with the expression of the collagen, a substrate thereof, an important involvement of HSP47 in various fibrosing diseases has been pointed out (patent documents 1-3, non-patent documents 6 and 8). In addition, a role of HSP47 in the fibrosing phase of acute lung injury and the like have also been reported (non-patent documents 1 - 3). Promoted expression of HSP47 in a topical lung tissue of lung fibrosing disease has been reported heretofore (non-patent documents 5 and 6). However, HSP47 has been considered to localize in the endoplasmic reticulum and not to leak extracellularly, increase of HSP47 outside the cell (for example, in the blood and the like) in interstitial pneumonia accompanied by fibrosing has not been expected. In fact, in the past reports, it was clear that the serum levels of HSP47 in patients with idiopathic pulmonary fibrosis were simillar to those of healthy individuals, and that no increase was observed in patients with idiopathic pulmonary fibrosis (non-patent document 7). It was not at all known or expected that HSP47 markedly increases in acute lung injury, and becomes a useful biomarker that can clearly distinguish acute lung injury from chronic progressive interstitial pneumonia.

Non-patent document 9 describes the utility of a panel of biomarkers for diagnosis and pathogenesis of acute lung injury in patients with traumatic injuries. Non-patent document 10 describes new target molecules ACE2, HMGB1 and HSP47 for research strategies for ARDS.

### [Document List]

### [patent documents]

patent document 1: JP-A-2008-122276
patent document 2: JP-A-2003-159087
patent document 3: JP-A-2002-522462

### [non-patent documents]

non-patent document 1: ICU & CCU, Vol. 33, No. 5, Page.351-359 (2009)
non-patent document 2: Surgical trauma & immunological responses, Vol. 17, No. 2, Page.62-64 (2008)
non-patent document 3: The Journal of Japan Society for Clinical Anesthesia, Vol. 23, No. 8, Page.S163 (2003)
non-patent document 4: Shuukan Igakuno Ayumi, Bessatsu (March) Page.134-136 (2003)
non-patent document 5: Hum Pathol 31:1498-1505(2000)
non-patent document 6: Respir Res 6:57(2005)
non-patent document 7: Biochem Biophys Res Commun 303:413-418 (2003)
non-patent document 8: Annals of the Japanese Respiratory Society, Vol. 40 extra number, Page.188 (2002)
non-patent document 9: Fremont et al., J. Trauma, Infection and Critical Care, Vol. 68, No.5, pages 1121-1127 non-patent document 10: Iwasaka, J. of Anethesia, Vol. 21, No.1, pages 122-123

### SUMMARY OF THE INVENTION

### Problems to be Solved by the Invention

An object of the present invention is to provide an examination method, and a diagnostic reagent useful for a diagnosis of acute lung injury by clearly distinguishing the same from chronic progressive interstitial pneumonia. Means of Solving the Problems

The present inventors have conducted intensive studies in an attempt to solve the aforementioned problems and found that the measurement of the HSP47 protein amount (concentration) in a biological sample is useful for a diagnosis of acute lung injury, and HSP47 protein in a biological sample specifically increases in acute lung injury as compared to chronic progressive interstitial pneumonia and the increase is useful for the distinction thereof, which resulted in the completion of the present invention.

That is, the present invention provides the following.
[1] A method of determining whether or not a test subject is affected with acute lung injury, comprising quantifying heat shock protein 47 in a biological sample derived from a test subject.
[2] The method of [1], which distinguishes acute lung injury from chronic progressive interstitial pneumonia.
[3] The method of [1] - [2], wherein the biological sample is blood, serum or plasma.
[4] Use of a reagent comprising an antibody that specifically recognizes heat shock protein 47 and/or collagen or a partial fragment thereof that has specific affinity for heat shock protein 47 for diagnosing acute lung injury *ex vivo.*
[5] A method of evaluating a treatment effect for or predicting the prognosis of acute lung injury of a test subject, comprising quantifying heat shock protein 47 in a biological sample derived from the test subject.

### Effect of the Invention

According to the examination method of the present invention, whether or not a test subject is affected with acute lung injury can be determined with high sensitivity. This method can be a powerful diagnostic tool capable of clearly distinguishing acute lung injury from chronic progressive interstitial pneumonia. Furthermore, using the diagnostic reagent of the present invention, whether or not being affected with acute lung injury can be conveniently diagnosed by the above-mentioned examination method of the present invention.

### Brief Description of the Drawings

Fig. 1 is a graph showing the concentration of HSP47 protein in the serum of healthy human and patients with each disease indicated on the horizontal axis.
Fig. 2 is a graph showing the concentration of KL-6 in the serum of healthy human and patients with each disease indicated on the horizontal axis.
Fig. 3 is a graph showing the concentration of SP-A in the serum of healthy human and patients with each disease indicated on the horizontal axis.
Fig. 4 is a graph showing the concentration of SP-D in the serum of healthy human and patients with each disease indicated on the horizontal axis.
Fig. 5 is a graph showing the concentration of LDH (lactate dehydrogenase) in the serum of healthy human and patients with each disease indicated on the horizontal axis.
Fig. 6 is a graph showing the relationship between the sensitivity and specificity in HSP47 protein, KL-6, SP-D, SP-A and LDH on a Receiver Operating Characteristic curve ((ROC) curve).
Fig. 7 is a graph showing the concentration of HSP47 protein in the serum of healthy human and patients with each disease indicated on the horizontal axis.
Fig. 8 is a graph showing the concentration of KL-6 in the serum of healthy human and patients with each disease indicated on the horizontal axis.
Fig. 9 is a graph showing the concentration of SP-A in the serum of healthy human and patients with each disease indicated on the horizontal axis.
Fig. 10 is a graph showing the concentration of SP-D in the serum of healthy human and patients with each disease indicated on the horizontal axis, wherein N.S. shows no significant difference.
Fig. 11 is a graph showing the concentration of LDH (lacticate dehydrogenase) in the serum of healthy human and patients with each disease indicated on the horizontal axis.
Fig. 12 is a graph showing the relationship between the sensitivity and specificity in HSP47 protein, KL-6, SP-D, SP-A and LDH on a Receiver Operating Characteristic curve. Description of Embodiments

The present invention is explained in detail in the following.

### (1) Use of heat shock protein 47 as a biomarker for the diagnosis of acute lung injury

The present invention provides a method for determining whether or not a test subject is affected with acute lung injury, comprising quantifying heat shock protein 47 in a biological sample derived from a test subject (method (1)).

In the present specification, the "acute lung injury (to be also referred to as ALI)" refers to an inflammatory disease of the lung, which is caused by sepsis, pneumonia, trauma and/or aspiration and the like that directly or indirectly injure the lung. The "acute lung injury" in the present specification also includes acute respiratory distress syndrome (to be also referred to as ARDS) which is known to be the same clinical disorder. Acute progressive interstitial pneumonia that meets the clinical definition of acute lung injury is also included in acute lung injury.

The clinical definition of acute lung injury is as described below (guideline for ALI/ARDS medical care; The Japanese Respiratory Society, Drafting Committee for ARDS Guideline):
(I) acute onset.
(II) hypoxia (PaO₂/FiO₂ is not more than 300 mmHg). (FiO₂=fraction of inspired O₂ concentration. PaO₂ unit is mmHg, FiO₂ is a decimal number (e.g., 0.5). A case showing PaO₂/FiO₂ of not more than 200 mmHg is acute respiratory distress syndrome.)
(III) Bilateral pulmonary infiltrative shadow is observed in chest radiography.
(IV) Pulmonary artery wedge pressure is not more than 18 mmHg or no physical clinical finding of elevation of left atrial pressure.

A case that satisfies all the above-mentioned criteria is defined as acute lung injury.

In the method (1) of the present invention, the "test subject" can be a "test subject suspected of being affected with acute lung injury". In the present specification, the "test subject suspected of being affected with acute lung injury" refers to a patient showing clinical findings (e.g., cough, expectoration, fever, respiratory distress, respiratory failure, hypoxia, chest pain etc.) common to acute lung injury, aortic dissection, acute heart failure, acute exacerbation of chronic heart failure, diffuse alveolar hemorrhage, lymphangitic carcinomatosis, reexpansion pulmonary edema, pulmonary edema by hyper-infusion, neurogenic pulmonary edema and lung infection (pulmonary tuberculosis, miliary tuberculosis etc.) by clinical diagnosis (these diseases other than acute lung injury are sometimes generically referred to as "the above-mentioned disease showing clinical finding common with acute lung injury" in the following).

In the method (1) of the present invention, the "test subject" or "test subject suspected of being affected with acute lung injury" can be a "test subject suspected of being affected with either acute lung injury or chronic progressive interstitial pneumonia". In the present specification, the "test subject suspected of being affected with either acute lung injury or chronic progressive interstitial pneumonia" refers to a patient who has a finding of suspected affection with either acute lung injury or chronic progressive interstitial pneumonia by diagnosis using chest radiography or a serum marker such as KL-6, SP-D, SP-A and the like.

In the present specification, the "interstitial pneumonia" refers to a disease with an inflammation principally in the interstitial tissue of the lung, which is characterized by alveolar septum hypertrophy, fibroblast growth, collagen deposition and the like. Examples of the interstitial pneumonia include those caused by radiation, infection with a pathogen, or a collagen disease, those induced by poisoning or drug, and idiopathic ones without clear etiology. Examples of the idiopathic interstitial pneumonia include, but are not limited to, cryptogenic organizing pneumonia (COP), idiopathic usual interstitial pneumonia (idiopathic UIP), idiopathic nonspecific interstitial pneumonia (idiopathic NSIP) and the like. In the present specification, the "interstitial pneumonia" is a concept also including lung fibrosis showing fibrotic inflammatory tissues due to progression.

A test subjects subjected to the method (1) of the present invention may be affected or non-affected with lung fibrosis. Although increased expression of HSP47 in a topical lung tissue of lung fibrosing disease has been reported heretofore (non-patent documents 5 and 6), since HSP47 has been considered to localize in the endoplasmic reticulum and not leak extracellularly, increase of HSP47 outside the cell (for example, in the blood and the like) in interstitial pneumonia accompanied by fibrosing has not been expected. In fact, in the past reports, the levels of HSP47 and anti-HSP47 antibody in the serum of patients with idiopathic pulmonary fibrosis were simillar to those of healthy individuals, and it was clear that no increase was observed (non-patent document 7). Extracellular (for example, in the blood and the like) HASP47 does not increase in chronic progressive interstitial pneumonia, but specifically increases in acute lung injury (including rapidly progressive interstitial pneumonia).

In a further aspect, in the method (1) of the present invention, the "test subject" and the "test subject suspected of being affected with acute lung injury" can be "a test subject suspected of being affected with acute lung injury or any of the above-mentioned disease showing a clinical finding common with acute lung injury". In the present specification, the "test subject suspected of being affected with acute lung injury or any of the above-mentioned disease showing a clinical finding common with acute lung injury" refers to a patient showing a clinical finding (e.g., respiratory distress, respiratory failure, hypoxia, chest pain etc.) common to acute lung injury and the above-mentioned disease by clinical diagnosis.

In the present specification, the "aortic dissection" refers to a disease with an acute chest pain or abdominal pain, which is developed by a breakage, due to genetic factors, degeneration and embrittlement of the arterial membrane, combined with arterial expansion, hypertension and the like, in a part of the intima of the arterial blood vessel that allows inflow of the blood into the intima from the hiatus of the intima to induce dissociation of the blood vessel wall.

In the present specification, the "acute heart failure" refers to the pathology of circulatory diseases wherein a sufficient blood flow is not supplied to the peripheral organs from the ventricle, due to rapid exacerbation of the circulation dynamics. Examples of the symptoms of the acute heart failure include marked respiratory distress based on lung congestion, cardiogenic shock based on low cardiac output, foamy expectoration, oliguria·anuria, coldness of the four limbs, lowering of blood pressure, cold sweat, tachycardia (sometimes bradycardiac) and the like.

In the present specification, the "acute exacerbation of chronic heart failure" refers to rapid exacerbation of chronic heart failure. In the present specification, the "chronic heart failure" refers to a pathology that gradually progresses as in obsolete myocardial infarction, dilated cardiomyopathy and the like, wherein various compensation mechanisms such as cardiac hypertrophy have acted to reach a certain equilibrium state, and shows symptoms of short breath, fatigability, lower motility tolerability, hepatosplenic swelling, edema, peripheral intravenous dilation and the like.

In the present specification, the "pulmonary tuberculosis" refers to lung infection with *Mycobacterium tuberculosis.*

In the present specification, the "miliary tuberculosis" refers to a pathology wherein a large amount of *Mycobacterium tuberculosis* enters into the blood circulation, diffuses in multiple organs to form many tubercles.

In the present specification, the "diffuse alveolar hemorrhage" refers to hemorrhage into the pulmonary alveolar cavity. Hemorrhage into the alveolar cavity, emergence of hemosiderin phagocyte (siderophore) in the alveolar cavity, and hemosiderin deposition on the alveolar wall are observed and, as a disease group showing diffuse disseminated shadow over the entire lung fields by chest radiography, alveolar hemorrhage syndrome is known. In many cases, hemorrhage is seen not only in the alveolar cavity but also lung stroma. The cause thereof includes, for example, lung damage, thoracic compression, blood vessel collapse in lung - bronchus lesion, blood vessel neurotic hemorrhage, partial hemorrhagic pneumonia, hemorrhage per diapedesis seen in blood diseases, hemorrhage due to microvessel disorder, hemorrhage related to immune phenomenon and the like.

In the present specification, the "lymphangitic carcinomatosis" refers to a condition wherein the cancer cells grown retrogradely due to the congestion of lymph flow by the metastasis of cancer cells into the lymph node on the perfusion side, embolism of lymphatic vessel and the like, spread with filling the lymphatic vessels of the tissue. The periphery of the expanded lymphatic vessel shows edema and growth of fiber tissues, and shows a pseudo-inflammatory state suggestive of lymphangitis. It is sometimes observed in breast cancer, gastric cancer, (metastatic) lung cancer and the like, and has bad prognosis.

In the present specification, the "pulmonary edema" refers to a pathological condition wherein abnormal water retention is observed outside the lung blood vessel.

In the present specification, the "reexpansion pulmonary edema" refers to pulmonary edema considered to be resulting from reperfusion of lung blood flow and blood vessel permeability enhancement caused by reexpansion of collapsed lung that occurs at once when pleural effusion, pneumothorax, hemothorax and the like are treated by thoracic cavity drainage and the like.

In the present specification, the "infusion" refers to a treatment method including administering water, electrolyte and the like by drip intravenous injection, wherein the term "pulmonary edema by hyper-infusion" refers to a state where the lung capillary pressure increases by excessive infusion to cause pulmonary edema.

In the present specification, the "neurogenic pulmonary edema" refers to acute pulmonary edema caused by increased intracranial pressure due to head trauma, epileptogenesis, cerebrovascular disorder and the like.

The test subject to be subjected to the method (1) of the present invention means a mammal. The mammal is not particularly limited as long as it has a possibility of being affected with acute lung injury, and experimental animals such as rodents (mouse, rat, hamster, guinea pig and the like), rabbit and the like, domestic animals such as swine, bovine, goat, horse, sheep, mink and the like, pets such as dog, cat and the like, primates such as human, monkey, Macaca fascicularis, Macaca mulatta, marmoset, orangutan, chimpanzee and the like are preferable, and human is particularly preferable.

Heat shock protein 47 (HSP47) is a known heat shock protein, and the amino acid sequence etc. thereof are also known. HSP47 used in the present invention is generally derived from a mammal. Being "derived from a mammal" means that the amino acid sequence of HSP47 is a mammalian sequence. The mammal may be the same as those usable as the test subjects. The kind of a mammal, from which HSP47 of the target to be quantified derives, is generally the same as the mammalian kind of the test subjects. For example, when the test subject is a human, human heat shock protein 47 is quantified.

Representative amino acid sequence of human HSP47 includes the amino acid sequence shown by SEQ ID NO: 2 (GeneBank accession No.:NP_001226). In the present specification, protein and peptides are described with the N-terminus (amino-terminus) on the left side, and the C-terminus (carboxyl-terminus) on the right side, according to conventional practice of indication.

Examples of the biological sample usable for the method (1) of the present invention include blood, broncho lavage fluid, alveolar lavage fluid, expectoration, lung tissue, brain spinal fluid, ascites and the like; however, it is not particularly limited as long as an increase in the HSP47 level associated with acute lung injury can be detected. Lung tissue is preferably disrupted in an appropriate buffer and the like and subjected to the method (1) of the present invention in the form of an extract. As the "blood", blood derived from any tissue can be assumed. Due to an easiness of its collection, peripheral blood is generally used. As a blood collection method, a method known per se can be applied. While the collected blood may be directly used for this step, it is preferably used for this step as a liquid component (plasma) separated from the cell components (red blood cell, leukocyte, platelet and the like) by method known per se, for example, centrifugation, filtration and the like. It is also preferable to coagulate the blood and use a liquid component (serum) separated from the platelets and coagulation factors for this step. The biological sample that can be used for the method (1) of the present invention is preferably blood, serum or plasma.

HSP47 in a biological sample can be quantified by a method known per se such as an immunological method, a mass spectrometry method and the like.

Quantification of HSP47 by an immunological method includes, for example, the following steps:
(1) contacting a biological sample derived from a test subject with an antibody that specifically recognizes HSP47 to form a complex of HSP47 in the biological sample and the antibody that specifically recognizes HSP47;
(2) detecting the complex formed in the above-mentioned step (1); and
(3) determining the amount of HSP47 in the biological sample from the amount of the complex detected in the above-mentioned step (2).

In step (1), a biological sample derived from a test subject is contacted with an antibody that specifically recognizes HSP47 to form a complex of HSP47 in the biological sample and the antibody that specifically recognizes HSP47.

In the present specification, the "antibody that specifically recognizes HSP47" only needs to have an ability to specifically bind HSP47, and may be any of polyclonal antibody and monoclonal antibody, and monoclonal antibody is preferable. Examples of the antibody include chimera antibody, single chain antibody or F(ab')2, Fab' of an antibody molecule or an affinity fragment such as Fab fraction and the like. As these antibodies, an antibody prepared by a method known per se and using HSP47 as an immunogen can be used, or a commercially available antibody can also be used.

Being "specific" means that the affinity for HSP47, which is an antigen of the antibody, is higher than the affinity for other antigens.

When recombinant HSP47 is used as the antigen, recombinant HSP47 can be produced, for example, by the following method. A polynucleotide encoding the amino acid sequence of HSP47 (e.g., in the case of human HSP47, polynucleotide containing the nucleotide sequence shown by SEQ ID NO: 1 (GeneBank accession No.: NN_001235)) is incorporated into an appropriate expression vector, the vector is introduced into an appropriate host for transformation, and the object recombinant HSP47 can be obtained from the homogenate of the transformed cell. The above-mentioned host cell is not particularly limited, and various host cells conventionally used for genetic engineering methods, for example, *Escherichia coli, Bacillus* species bacteria, yeast, plant or animal cell and the like can be used.

HSP47 may be, in addition to a recombinant protein produced from the above-mentioned transformant, one isolated or purified from a natural cell producing same, by a protein separation and purification technique known per se. Moreover, it may be a protein synthesized by a chemical synthesis or synthesized biochemically in cell-free translation system.

Using the above-mentioned HSP47, the aforementioned "antibody that specifically recognizes HSP47" can be produced according to a conventional method. Specifically, when the antibody is a polyclonal antibody, a non-human animal such as rabbit and the like is immunized with HSP47 according to a conventional method, and the antibody can be obtained from serum of the immunized animal (Current Protocols in Molecular Biology, edit. Ausubel FM et al. (2011) Publish. John Wiley and Sons. Chapter 11, Section III, Unit 11.12 - 11.13). On the other hand, in the case of a monoclonal antibody, a non-human animal such as mouse and the like is immunized with HSP47 or an oligopeptide having a partial amino acid sequence thereof and the like according to a conventional method, the obtained spleen cell and a myeloma cell are fused, and the antibody can be obtained from the prepared hybridoma cell (Current protocols in Molecular Biology, edit. Ausubel FM et al. (2011) Publish. John Wiley and Sons. Chapter 11, Section II, Unit 11.4 - 11.11).

The antibody that specifically recognizes HSP47, which is to be used in the present invention, is preferably isolated or purified. Being "isolated or purified" means that an operation to remove components other than the object component from the natural state has been applied. The purity of the isolated or purified antibody that specifically recognizes HSP47 (weight ratio of antibody that specifically recognizes HSP47 relative to total protein weight) is generally not less than 50%, preferably not less than 70%, more preferably not less than 90%, most preferably not less than 95% (for example, substantially 100%).

The aforementioned antibody may be directly or indirectly labeled with a labeling substance. Examples of the labeling substance include fluorescent substance (e.g., FITC, rhodamine), radioactive substance (e.g., ¹⁴C, ³H, ¹²⁵I), enzyme (e.g., alkaline phosphatase, peroxidase), colored particles (e.g., colloidal metal particles, colored latex), biotin and the like.

Such antibody may be used alone or two or more kinds thereof may be used in this step.

The above-mentioned "antibody that specifically recognizes HSP47" can be used in the form of an aqueous solution; however, it is preferably bound to a solid phase. Examples of such "solid phase" include a plate (e.g., a microwell plate), a tube, beads (e.g., plastic beads, magnetic beads), a chromatography carrier (e.g., Sepharose (trade mark)), a membrane (e.g., a nitrocellulose membrane, a PVDF membrane), a gel (e.g., a polyacrylamide gel), a metal film (e.g., a gold film) and the like. Of these, plate, beads, membrane and metal film are preferably used, and a plate is most preferably used in view of the convenience of handling. The above-mentioned bond is not particularly limited and covalent bond, ionic bond, physical adsorption and the like can be mentioned. Covalent bond and/or physical adsorption are/is preferable for achieving sufficient bond strength. For binding to a solid phase, it may be directly binding to a solid phase or indirectly binding to a solid phase by utilizing a substance known per se. Furthermore, to suppress non-specific adsorption and non-specific reaction, a phosphate buffered solution of bovine serum albumin (BSA), cow's milk protein and the like is contacted with a solid phase, and a solid phase surface not coated with the antibody is generally blocked with the aforementioned BSA, cow's milk protein and the like.

In this step, the contact between the "antibody that specifically recognizes HSP47" and the "HSP47" contained in a biological sample derived from a test subject is carried out by mixing said biological sample and the antibody that specifically recognizes HSP47 in a reaction vessel and, as long as the method permits their interaction, the embodiment, order, specific method and the like are not particularly limited. The contact is performed by, for example, adding (extract of) said biological sample to a plate on which the "antibody that specifically recognizes HSP47" is immobilized.

The time of maintaining such contact is not particularly limited as long as the aforementioned antibody that specifically recognizes HSP47 can bind to HSP47 contained in a biological sample derived from a test subject to form a complex, and it is generally several seconds to more than a dozen hours. To rapidly determine whether acute lung injury or not, it is preferably 1 min to 2 hr, most preferably 2 min to 30 min. The temperature condition of contact is generally 4°C - 50°C, preferably 4°C - 37°C, most preferably room temperature of about 15°C - 30°C. Furthermore, the pH condition for the reaction is preferably 5.0 - 9.0, particularly preferably the neutral range of 6.0 - 8.0.

In step (2), the complex formed in the above-mentioned step (1) is detected to determine whether HSP47 is present or not in the above-mentioned biological sample.

The above-mentioned detection is performed by detecting "HSP47" or "antibody that specifically recognizes HSP47" contained in the complex. For the detection, enzyme immunoassay (EIA method), immunochromatography, latex agglutination, radioimmunoassay (RIA method), fluorescent immunoassay (FIA method), luminescence immunoassay, surface plasmon resonance measurement method (SPR method) and the like can be utilized. Of these, EIA method, immunochromatography, FIA method and SPR method are preferable from the aspects of easiness and rapidness of the operation.

When EIA method is selected as the detection method in step (2), the EIA method is preferably sandwich Enzyme-Linked ImmunoSorbent Assay (sandwich ELISA method) using two kinds of the "antibody that specifically recognizes HSP47". Since two kinds of antibodies are used in the sandwich ELISA method, it is superior in the specificity to antigen.

The two kinds of "antibodies that specifically recognize HSP47" for performing the Sandwich ELISA method may be any combination of monoclonal antibodies, polyclonal antibodies, or monoclonal antibody and polyclonal antibody, as long as the epitopes are noncompetitive.

As one kind of Sandwich ELISA method, a method utilizing an avidin-biotin reaction is applicable. In this method, for example, HSP47 in the plasma or serum is trapped by any immobilized "antibody that specifically recognizes HSP47", and an antigen-antibody reaction is performed between the trapped HSP47 and biotin-labeled "antibody that specifically recognizes HSP47". The time required for such reaction is preferably 1 min - 2 hr, more preferably 2 min - 30 min, since a rapid measurement is necessary. Then, enzyme-labeled streptavidin is added to perform avidin-biotin reaction. The time required for such reaction is preferably 5 min - 1 hr, more preferably 15 min - 30 min, since a rapid measurement is necessary. Then HSP47 is specifically detected by detecting the enzyme. The above-mentioned biotin-labeled "antibody that specifically recognizes HSP47" can be produced by binding biotin to an "antibody that specifically recognizes HSP47" by a method known per se. For example, using a commercially available biotin labeling kit, biotin and "antibody that specifically recognizes HSP47" can be bonded. As the enzyme-labeled streptavidin, a commercially available product can be preferably used.

Also, sandwich ELISA method utilizing an enzyme-labeled antibody is also applicable. In this method, for example, HSP47 in the blood is trapped by any immobilized "antibody that specifically recognizes HSP47", and an antigen-antibody reaction is performed between the trapped HSP47 and enzyme-labeled "antibody that specifically recognizes HSP47". The time required for such reaction is preferably 1 min - 2 hr, more preferably 2 min - 30 min, since a rapid measurement is necessary. Then HSP47 is detected by detecting the enzyme. The enzyme-labeled antibody can be produced by binding(labeling) the enzyme to "antibody that specifically recognizes HSP47" by a method known per se, for example, glutaraldehyde method, maleimide method and the like.

Furthermore, from the aspects of broad utility, sandwich ELISA method utilizing a secondary antibody is also applicable. In this method, for example, HSP47 in blood is trapped by any immobilized "antibody that specifically recognizes HSP47", and an antigen-antibody reaction is performed between the trapped HSP47 and "antibody that specifically recognizes HSP47" which is derived from an animal species different from that for the immobilized antibody (indicated as primary antibody in this paragraph). The time required for such reaction is preferably 1 min - 2 hr, more preferably 2 min - 30 min, since a rapid measurement is necessary. For example, when the immobilized "antibody that specifically recognizes HSP47" is derived from rabbit, the reaction is performed using a primary antibody derived from an animal species other than rabbit, for example, an antibody derived from mouse. Then, an antigen-antibody reaction is performed between the primary antibody and the enzyme-labeled "antibody recognizing the primary antibody" (indicated as secondary antibody in this paragraph). The time required for such reaction is preferably 1 min - 2 hr, more preferably 2 min - 30 min, since a rapid measurement is necessary. Finally, HSP47 is detected by detecting the enzyme. As the enzyme-labeled secondary antibody, a commercially available product can be preferably used.

As the "enzyme" of the enzyme-labeled streptavidin and enzyme-labeled antibody, peroxidase, alkaline phosphatase, glucose oxidase, β-galactosidase and the like are exemplified.

As a substrate reagent to be used for the detection of the enzyme, one suitable for the selected labeling enzyme is used. For example, when a peroxidase is selected as an enzyme, o-phenylenediamine (OPD), tetramethylbenzidine (TMB) and the like are used. When an alkaliphosphatase is selected, p-nitrophenyl phosphate (PNPP) and the like are used. As a reaction quenching liquid and a substrate dissolution liquid, conventionally known ones can be used as appropriate according to the selected enzyme, without any particular limitation.

Even when sandwich ELISA method is not used, detection is possible by applying a general ELISA method. For example, in step (1), HSP47 in the blood derived from a test subject is bound to a solid phase in the same manner as in the aforementioned method, then an antigen-antibody reaction is performed with the labeled "antibody that specifically recognizes HSP47" to form a complex. The time required for such reaction is preferably 1 min - 2 hr, more preferably 2 min - 30 min, since a rapid measurement is necessary. Then HSP47 can be detected by using a method according to the label.

When immunochromatography is selected as a detection method in step (2), developing of blood from under a membrane, for example, allows HSP47 to be trapped by an "antibody that specifically recognizes HSP47" immobilized, in a line form, on a water-absorbing substrate such as nitrocellulose membrane and the like, and an antigen-antibody reaction is performed between the trapped HSP47 and the labeled "antibody that specifically recognizes HSP47". The time required for such reaction is preferably 1 min - 2 hr, more preferably 2 min - 30 min, since a rapid measurement is necessary. Then HSP47 can be detected by using a method according to the label.

The two kinds of "antibodies that specifically recognize HSP47" for performing immunochromatography may also be any combination of monoclonal antibodies, polyclonal antibodies, or monoclonal antibody and polyclonal antibody, as long as the epitopes are noncompetitive.

When FIA method is selected as a detection method in step (2), the enzyme bound to the "antibody that specifically recognizes HSP47" used for the above-mentioned EIA method is substituted for a fluorescent substance and the obtained antibody is used to perform sandwich ELISA in the same manner as in the above-mentioned method. Then, the fluorescent substance is detected using a commercially available measurement device, fluorescence microscope, confocal microscope and the like, whereby HSP47 is detected. As the fluorescent substance, chemical substances such as APC, PE, Cy2, Cy3, Cy5, ECD, FITC, PerCP, Alexa (registered trade mark) Fluor, fluorescein, rhodamine and the like can be preferably utilized. The chemical substance can be labeled to an antibody by a method known per se.

When SPR method is selected as a detection method in step (2), an interaction between an antibody that specifically recognizes HSP47, which is immobilized in advance on a surface of a metal film (sensor chip), and HSP47 in blood, which is flown on a surface of the metal film is detected as time course changes of the surface plasmon resonance. When the SPR method is performed, the "antibody that specifically recognizes HSP47" which is immobilized on a metal film to be a sensor chip may be only one kind, and may be a monoclonal antibody or a polyclonal antibody. As a measurement device utilized for the detection of SPR, a commercially available measurement device can be preferably used.

In one specific example, when EIA method is applied, the blood collected from a test subject is shaken for a predetermined time at room temperature, and centrifuged to give serum. Then the serum is dispensed into a microplate immobilizing any "antibody that specifically recognizes HSP47", and left standing at room temperature for a predetermined time. After the plate is washed to remove unreacted antigen, the biotinylated, above-mentioned antibody solution is dispensed into a plate and left standing for a predetermined time to form a complex. The plate is further washed to remove unreacted antibody, peroxidase labeled streptavidin solution is dispensed into a plate and the mixture is reacted at room temperature for a predetermined time. The plate is washed, and reacted with a chromogen solution such as TMB and the like to detect the complex.

In another specific example, when immunochromatography is applied, a test piece is immersed in the above-mentioned serum or serum diluted with saline and the like to allow development. In the test piece, carrier maintaining a particulate labeled substance, and a filter paper used for an absorption carrier of a liquid sample are laminated via one end on the lower end side of a strip-shaped antibody immobilized support; on the other hand, a liquid-absorbing carrier made of a filter paper is laminated via one end on the upper end side of the aforementioned antibody immobilized support. The above-mentioned antibody immobilized support comprises a nitrocellulose sheet and an "antibody that specifically recognizes HSP47", which performs an antigen-antibody reaction with HSP47, immobilized on the sheet. Immobilization is performed by applying the above-mentioned antibody solution on the nitrocellulose sheet and drying it. The immobilized antibody specifically recognizes HSP47 in the developed serum and can trap a complex of HSP47 and the above-mentioned particulate-labeled antibody. Therefore, when the "antibody that specifically recognizes HSP47" is, in a line form, immobilized on the antibody-immobilized support, the line is colored by the particulate label to determine the presence of HSP47 in the blood.

In step (3), the amount of HSP47 in a biological sample is determined from the amount of the complex detected in the above-mentioned step (2).

The amount (concentration) of the complex detected in the above-mentioned step (2) can be measured by correlating a signal of fluorescence intensity or absorbance and the like to a separately prepared calibration curve. The concentration of HSP47 in a biological sample of a test subject can be quantified by multiplying the concentration obtained from the calibration curve by the dilution rate.

The calibration curve is shown by an approximation formula plotting the measured values, wherein the horizontal axis (vertical axis) shows the concentration of the samples obtained by serially diluting the aforementioned standard HSP47 protein standard product, and the vertical axis (horizontal axis) shows the signal (in any unit) of the antibody that specifically recognizes HSP47, which is bound to HSP47 in a standard HSP47 protein. The calibration curve can be shown in an approximate straight line by logarithmic presentation of the both axes.

Next, the amount of HSP47 in a biological sample derived from a test subject is correlated with the possibility of being affected with acute lung injury, and whether or not the test subject is affected with acute lung injury is determined based on the correlation. As shown in the below-mentioned Examples, as compared to healthy human and patients with chronic progressive interstitial pneumonia, patients with acute lung injury show a higher amount of HSP47 in a biological sample. Moreover, as compared to patients with the above-mentioned disease that shows clinical findings common with acute lung injury, patients with acute lung injury show a higher amount of HSP47 in a biological sample. The above-mentioned determination is made based on the positive correlation between such amount of HSP47 in a biological sample derived from a test subject and the possibility of being affected with acute lung injury.

For example, biological samples (e.g., serum) are collected from healthy human or patients with chronic progressive interstitial pneumonia (negative control), and patients with acute lung injury (positive control), the amount of HSP47 in the biological samples collected from the test subjects is compared with that of the positive control and negative control. Alternatively, a correlation diagram of the amount of HSP47 in a particular biological sample (e.g., serum) and acute lung injury morbidity rate is prepared in advance, and the amount of HSP47 in a biological sample collected from a test subject may be compared with the correlation diagram. The amount of HSP47 is preferably compared based on the presence or absence of a significant difference.

From the comparison results of the amount of HSP47, when the amount of HSP47 in a biological sample derived from a test subject is relatively high, the test subject can be determined to have a relatively high possibility of being affected with acute lung injury. Conversely, when the amount of HSP47 in a biological sample derived from a test subject is relatively low, the test subject can be determined to have a relatively low possibility of being affected with acute lung injury. In a differentiation of acute lung injury from chronic progressive interstitial pneumonia using the method (1) of the present invention, when the amount of HSP47 in a biological sample derived from a test subject is relatively high, the test subject can be determined to have a relatively high possibility of being affected with acute lung injury rather than chronic progressive interstitial pneumonia. Conversely, when the amount of HSP47 in a biological sample derived from a test subject is relatively low, the test subject can be determined to have a high possibility of being affected with chronic progressive interstitial pneumonia rather than acute lung injury.

Alternatively, a cutoff value of the amount of HSP47 in a biological sample may be determined in advance, and the cutoff value may be compared with the measured amount of HSP47. For example, when the amount of HSP47 in a biological sample derived from a test subject is not less than the aforementioned cutoff value, the test subject can be determined to have a high possibility of being affected with acute lung injury. Conversely, when the amount of HSP47 in a biological sample derived from a test subject is lower than the cutoff value, the test subject can be determined to have a low possibility of being affected with acute lung injury. In a differentiation of acute lung injury from chronic progressive interstitial pneumonia using the method (1) of the present invention, when the amount of HSP47 in a biological sample derived from a test subject is not less than the aforementioned cutoff value, the test subject can be determined to have a relatively high possibility of being affected with acute lung injury rather than chronic progressive interstitial pneumonia. Conversely, when the amount of HSP47 in a biological sample derived from a test subject is lower than the cutoff value, the test subject can be determined to have a high possibility of being affected with chronic progressive interstitial pneumonia rather than acute lung injury.

The "cutoff value" can satisfy both high diagnostic sensitivity (true positive rate) and high diagnosis specificity (true negative rate), when the disease and condition are evaluated using the value as a standard. For example, an amount of HSP47 that shows high positive rate in patients with acute lung injury and high negative rate in healthy individual or patients with chronic progressive interstitial pneumonia or other respiratory diseases can be set as a cutoff value. For example, when whether or not a test subject is affected with acute lung injury is determined using peripheral blood serum as a biological sample, a preferable cutoff value is 100 - 1500 pg/ml, preferably 200 - 1300 pg/ml, more preferably 300 - 1200 pg/ml, further more preferably 400 - 1000 pg/ml, most preferably 500 - 900 pg/ml. In addition, when acute lung injury is distinguished from chronic progressive interstitial pneumonia by using peripheral blood serum as a biological sample, a preferable cutoff value is 100 - 1500 pg/ml, preferably 200 - 1300 pg/ml, more preferably 300 - 1200 pg/ml, further more preferably 400 - 1000 pg/ml, most preferably 500 - 900 pg/ml.

In a further aspect, acute lung injury can be distinguished from the above-mentioned diseases showing clinical findings common with acute lung injury by using the method (1) of the present invention. Clinical findings of acute lung injury are similar to those of the above-mentioned diseases. Since the above-mentioned diseases showing clinical findings common with acute lung injury do not have a factor that promotes collagen synthesis, the amount of HSP47 in the biological sample does not increase.

Specifically, for example, biological samples (e.g., serum) are collected from healthy human or patients with the above-mentioned diseases showing clinical findings common with acute lung injury (negative control), and patients with acute lung injury (positive control), the amount of HSP47 in the biological samples collected from the test subjects is compared with that of the positive control and negative control. Alternatively, a correlation diagram between the amount of HSP47 in a particular biological sample (e.g., serum) and acute lung injury morbidity rate is prepared in advance, and the amount of HSP47 in a biological sample collected from a test subject may be compared with the correlation diagram. The amount of HSP47 is preferably compared based on the presence or absence of a significant difference.

When the amount of HSP47 in a biological sample derived from a test subject is relatively high, the test subject can be determined to have a relatively high possibility of being affected with acute lung injury rather than the above-mentioned diseases showing clinical findings common with acute lung injury. Conversely, when the amount of HSP47 in a biological sample derived from a test subject is relatively low, the test subject can be determined to have a high possibility of being affected with the above-mentioned diseases showing clinical findings common with acute lung injury rather than acute lung injury.

Moreover, the present invention provides a diagnostic reagent for acute lung injury comprising the aforementioned antibody that specifically recognizes HSP47. Said diagnostic reagent can be a diagnosis kit for acute lung injury. Using the diagnostic reagent of the present invention, whether or not a test subject is affected with acute lung injury can be easily determined by quantifying HSP47 in a biological sample derived from a test subject by an immunological means in the aforementioned method (1) of the present invention, or acute lung injury can be distinguished from chronic progressive interstitial pneumonia or the above-mentioned diseases showing clinical findings common with acute lung injury.

The antibody may be directly or indirectly labeled with a labeling substance. Examples of the labeling substance include a fluorescent substance (e.g., FITC, rhodamine), a radioactive substance (e.g., ¹⁴C, ³H, ¹²⁵I), an enzyme (e.g., an alkaline phosphatase, a peroxidase), a colored particle (e.g., a colloidal metal particle, a colored latex), biotin and the like.

The antibody can also be used in the state of an aqueous solution, and it is preferably bound to a solid phase. Examples of the "solid phase" include a plate (e.g., a microwell plate), a tube, beads (e.g., plastic beads, magnetic beads), a chromatography carrier (e.g., Sepharose (trade mark)), a membrane (e.g., a nitrocellulose membrane, a PVDF membrane), a gel (e.g., a polyacrylamide gel), a metal film (e.g., a gold film) and the like.

The diagnostic reagent of the present invention may comprise, besides an antibody that specifically recognizes HSP47, a reagent to be used for quantifying the amount of HSP47 in a biological sample and the like. These reagents may be stored in advance together with the antibody that specifically recognizes HSP47, or stored in separate containers. Examples of the reagent include a treatment liquid, a buffer for diluting an antibody, a secondary antibody, a labeling substance (e.g., a fluorescence dye, an enzyme), a reaction vessel, a positive control (e.g., a recombinant HSP47), a negative control, a solid phase, an instruction sheet describing the examination protocol and the like. These components can also be mixed in advance as necessary.

In addition, since collagen has a property of specifically binding to HSP47, "collagen or a partial fragment thereof having specific affinity for HSP47" may be used instead of or in addition to the "antibody that specifically recognizes HSP47" for the quantification of HSP47 by the method of the present invention in another embodiment.

Quantification of HSP47 by a method using collagen or a partial fragment thereof having specific affinity for HSP47 (the method (2) of the present invention) includes, for example, the following steps:
(1') contacting a biological sample derived from a test subject with collagen or a partial fragment thereof having specific affinity for HSP47, to form a complex of HSP47 in the biological sample and the collagen or a partial fragment thereof having specific affinity for HSP47;
(2') detecting the complex formed in the above-mentioned step (1); and
(3') determining the amount of HSP47 in the biological sample from the amount of the complex detected in the above-mentioned step (2).

In step (1'), a biological sample derived from a test subject is contacted with collagen or a partial fragment thereof having specific affinity for HSP47 to form a complex of HSP47 in the biological sample and the collagen or a partial fragment thereof having specific affinity for HSP47.

The collagen to be used for the method of the present invention is mammalian collagen. Examples of the mammal include, but are not limited to, experimental animals such as rodents (mouse, rat, hamster, guinea pig and the like), rabbit and the like, domestic animals such as swine, bovine, goat, horse, sheep, mink and the like, pets such as dog, cat and the like, primates such as human, monkey, *Macaca fascicularis, Macaca mulatta,* marmoset, orangutan, chimpanzee and the like. The collagen to be used for the method of the present invention is preferably collagen of the same species of mammal as the test subject. For example, when the test subject is human, human collagen is preferably used.

In the present specification, the "human collagen" means that the amino acid sequence of collagen is the same as that of the collagen naturally expressed in human.

The collagen to be used for the method of the present invention may be of any type as long as it has a property of specifically binding to HSP47. Mammalian collagen encompasses 27 types of types I - XXVII. The mammalian collagen is preferably types I - V, more preferably type I. It is known that at least mammalian collagens of types I - V bind to HSP47 (Natsume T. et al., J. Biol. Chem., 269, 31224-31228, 1994).

The collagen is generally a triple helix containing 3 polypeptides. For example, type I-collagen is a triple helix containing two α1 chains (type I) and one α2 chain (type I). A representative amino acid sequence of the α1 chain of human type I-collagen is described in SEQ ID NO: 4, and a representative amino acid sequence of the α2 chain of human type I-collagen is described in SEQ ID NO: 6.

The "partial fragment of collagen having specific affinity for HSP47" may be a partial fragment of any region of collagen as long as it has specific affinity for HSP47. The partial fragment of collagen having specific affinity for HSP47 is generally a triple helix containing 3 partial peptides (when 3 polypeptides constituting collagen are polypeptides A, B and C, respective partial peptides are a fragment of polypeptide A, a fragment of polypeptide B, and a fragment of polypeptide C).

The size of the three partial peptides contained in the "partial fragment of collagen having specific affinity for HSP47" is generally not less than 9 amino acids, preferably not less than 12 amino acids, more preferably not less than 15 amino acids, further more preferably not less than 18 amino acids.

The 3 partial peptides contained in the "partial fragment of collagen having specific affinity for HSP47" respectively contain, for example, not less than 4, preferably not less than 5, more preferably not less than 6, Gly-Xaa-Yaa (wherein Xaa and Yaa are any amino acids) repeats contained in the polypeptide constituting collagen.

To secure specific affinity for HSP47, the partial fragment preferably contains at least one arginine residue in the Yaa moiety per one fragment (Koide, T. et al., J. Biol. Chem., 281, 3432-3438, 2006).

Furthermore, to secure specific affinity for HSP47, at least one partial peptide contained in the "partial fragment of collagen having specific affinity for HSP47" preferably is at least one amino acid sequence shown by Yaa⁻³-Gly-Xaa⁻¹-Arg-Gly wherein Xaa⁻¹ is any amino acid (preferably Pro), and Yaa⁻³ is any amino acid (preferably Thr, Pro, Ser, Hyp, Val, Ala, Ile, Leu, Asn, Met, His, Phe or Tyr, more preferably Thr, Pro, Ser, Hyp, Val or Ala, further preferably Thr or Pro, most preferably Thr). In one preferable embodiment, Yaa⁻³-Gly-Xaa⁻¹-Arg-Gly is Thr-Gly-Pro-Arg-Gly (see Koide T. et al., J. Biol. Chem. 281, 11177-11185, 2006).

In the present specification, "any amino acid" encompasses Ala, Cys, Asp, Glu, Phe, Gly, His, Ile, Lys, Leu, Met, Asn, Pro, Gln, Arg, Ser, Thr, Val, Trp, Tyr and Hyp.

The collagen and a partial fragment thereof that specifically bind to HSP47, and the amino acid sequence in collagen significant for specific binding to HSP47 are described in detail in Koide, T. et al., J. Biol. Chem. 277, 6178-6182, 2002; Tasab, M. et al., J. Biol. Chem. 277, 35007-35012, 2002; Koide, T., et al., J. Biol. Chem. 281, 3432-3438, 2006; and Natsume, T. et al., J. Biol. Chem. 269, 31224-31228, 1994, which are incorporated in their entireties in the present specification by reference, and those of ordinary skill in the art can easily obtain collagen specifically binding to HSP47 and a partial fragment thereof, based on the description of the present specification, those documents and the like.

The collagen or a partial fragment thereof having specific affinity for HSP47, which is used for quantification of HSP47, may contain, in addition to the amino acid sequence derived from the collagen itself, 1 or more (e.g., 1 - 500, preferably about 1 - 100, more preferably about 1-15) additional amino acids. Such addition of amino acid is acceptable as long as the collagen or a partial fragment thereof having specific affinity for HSP47 specifically recognizes HSP47. The amino acid sequence to be added is not particularly limited, and examples include first methionone, cysteine and a tag that facilitates detection and purification of polypeptide, and the like. Examples of the tag include Flag tag, histidine tag, c-Myc tag, HA tag, AU1 tag, GST tag, MBP tag, fluorescent protein tag (e.g., GFP, YFP, RFP, CFP, BFP etc.), immunoglobulin Fc tag and the like. The position of the amino acid sequence addition is preferably N-terminus or C-terminus of polypeptide.

The collagen or a partial fragment thereof having specific affinity for HSP47 to be used in the present invention is preferably isolated or purified. Being "isolated or purified" means that an operation to remove components other than the object component from the natural state has been applied. The purity of the isolated or purified collagen or a partial fragment thereof having specific affinity for HSP47 (weight ratio of collagen or a partial fragment thereof having specific affinity for HSP47 relative to total protein weight) is generally not less than 50%, preferably not less than 70%, more preferably not less than 90%, most preferably not less than 95% (for example, substantially 100%).

The amino group of the amino acid residue at the N-terminus and C-terminus of the collagen or a partial fragment thereof having specific affinity for HSP47 to be used for the method of the present invention may be protected by a protecting group (e.g., a formyl group, a C1-6 acyl group such as C1-6 alkanoyl (an acetyl group etc.) and the like).

The aforementioned collagen or a partial fragment thereof having specific affinity for HSP47 may be directly or indirectly labeled with a labeling substance. Examples of the labeling substance include fluorescent substance (e.g., FITC, rhodamine), radioactive substance (e.g., ¹⁴C, ³H, ¹²⁵I); enzyme (e.g., alkaline phosphatase, peroxidase), colored particles (e.g., colloidal metal particles, colored latex), biotin and the like.

As such collagen or a partial fragment thereof having specific affinity for HSP47 in this step, only one kind of collagen or a partial fragment thereof having specific affinity for HSP47, or two or more kinds thereof may be used.

When recombinant collagen is used for the quantification of HSP47, recombinant collagen can be produced, for example, by the following method. A polynucleotide encoding the collagen subunit amino acid sequence (e.g., in the case of human Type 1 collagen, polynucleotide containing nucleotide sequences shown by SEQ ID NO: 3 and SEQ ID NO: 5 (GeneBank accession Nos.: NM_000088.3 and NM_000089.3)) is incorporated into an appropriate expression vector, the vector is introduced into an appropriate host for transformation and association of collagen subunit molecules in the host, and the object recombinant collagen can be obtained from the culture supernatant of the transformant. The above-mentioned host cell is not particularly limited, and various host cells conventionally used for genetic engineering methods, for example, *Escherichia coli, Bacillus* species bacteria, yeast, plant or animal cell and the like can be used.

Collagen may be, in addition to a recombinant protein produced from the above-mentioned transformant, one isolated or purified from a natural cell producing it or culture supernatant thereof, by a protein separation and purification technique known per se. Moreover, it may be a protein synthesized by a chemical synthesis or synthesized biochemically in cell-free translation system.

The above-mentioned "collagen or a partial fragment thereof having specific affinity for HSP47" can be used in the form of an aqueous solution; however, it is preferably bound to a solid phase. Examples of such "solid phase" include a plate (e.g., a microwell plate), a tube, beads (e.g., plastic beads, magnetic beads), a chromatography carrier (e.g., Sepharose (trade mark)), a membrane (e.g., a nitrocellulose membrane, a PVDF membrane), a gel (e.g., a polyacrylamide gel), a metal film (e.g., a gold film) and the like. Of these, a plate, beads, a membrane and a metal film are preferably used, and a plate is most preferably used in view of the convenience of handling. The above-mentioned bond is not particularly limited and covalent bond, ionic bond, physical adsorption and the like can be mentioned. Covalent bond and/or physical adsorption are/is preferable for achieving sufficient bond strength. As a binding to a solid phase, it may be directly bound to a solid phase or indirectly bound to a solid phase by utilizing a substance known per se. Furthermore, to suppress non-specific adsorption and non-specific reaction, a phosphate buffered solution of bovine serum albumin (BSA), cow's milk protein and the like is contacted with a solid phase, and a solid phase surface not coated with the antibody is generally blocked with the aforementioned BSA, cow's milk protein and the like.

In this step, the contact between the "collagen or a partial fragment thereof having specific affinity for HSP47" and the "HSP47" contained in a biological sample derived from a test subject is carried out by mixing said biological sample and the collagen or a partial fragment thereof having specific affinity for HSP47 in a reaction container and, as long as the method permits their interaction, the embodiment, order, specific method and the like are not particularly limited. The contact is performed by, for example, adding (extract of) said biological sample to a plate on which the "collagen or a partial fragment thereof having specific affinity for HSP47" is immobilized.

The time of maintaining such contact is not particularly limited as long as it is sufficient for the aforementioned collagen or a partial fragment thereof having specific affinity for HSP47 can bind to HSP47 contained in a biological sample derived from a test subject to form a complex, and it is generally several seconds to more than a dozen hours. To rapidly determine whether acute lung injury or not, it is preferably 1 min to 2 hr, most preferably 2 min to 30 min. The temperature condition of contact is generally 4°C - 50°C, preferably 4°C - 37°C, most preferably room temperature of about 15°C - 30°C. Furthermore, the pH condition for the reaction is preferably 5.0 - 9.0, particularly preferably the neutral range of 6.0 - 8.0.

In step (2'), the complex formed in the above-mentioned step (1') is detected to determine whether HSP47 is present or not in the above-mentioned biological sample.

The above-mentioned detection is performed by detecting "HSP47" contained in the complex. For the detection, enzyme immunoassay (EIA method), immunochromatography, latex agglutination, radioimmunoassay (RIA method), fluorescent immunoassay (FIA method), luminescence immunoassay, surface plasmon resonance measurement method (SPR method) and the like can be utilized. Of these, EIA method, immunochromatography, FIA method and SPR method are preferable from the aspects of easiness and rapidness of the operation.

When EIA method is selected as the detection method in step (2'), the EIA method is preferably ELISA method.

As one kind of ELISA method, ELISA method utilizing an enzyme-labeled antibody is applicable. In this method, for example, HSP47 in the blood is trapped by any immobilized "collagen or a partial fragment thereof having specific affinity for HSP47", and an antigen-antibody reaction is performed between the trapped HSP47 and enzyme-labeled "antibody that specifically recognizes HSP47". The time required for such reaction is preferably 1 min - 2 hr, more preferably 2 min - 30 min, since a rapid measurement is necessary. Then HSP47 is detected by detecting the enzyme. The enzyme-labeled antibody can be produced by binding(labeling) the enzyme to "antibody that specifically recognizes HSP47" by a method known per se, for example, glutaraldehyde method, maleimide method and the like.

The definition of the "antibody that specifically recognizes HSP47" is as mentioned above.

As the "enzyme" of the enzyme-labeled antibody, peroxidase, alkaline phosphatase, glucose oxidase, β-galactosidase and the like are exemplified.

As a substrate reagent to be used for the detection of the enzyme, one suitable for the selected labeling enzyme is used. For example, when a peroxidase is selected as an enzyme, o-phenylenediamine (OPD), tetramethylbenzidine (TMB) and the like are used. When an alkaliphosphatase is selected, p-nitrophenyl phosphate (PNPP) and the like are used. As a reaction quenching liquid and a substrate dissolution liquid, conventionally known ones can be used as appropriate according to the selected enzyme, without any particular limitation.

In one specific example, when the above-mentioned EIA method is applied, the blood collected from a test subject is shaken for a predetermined time at room temperature, and centrifuged to give serum. Then the serum is dispensed into a microplate immobilizing any "collagen or a partial fragment thereof having specific affinity for HSP47", and left standing at room temperature for a predetermined time. After the plate is washed to remove unreacted antibody, a solution of the antibody that specifically recognizes HSP47, which is labeled with peroxidase etc. is dispensed into a plate and the mixture is reacted at room temperature for a predetermined time. The plate is washed, and reacted with a solution of chromogen, such as TMB and the like to detect the complex.

In step (3'), the amount of HSP47 in a biological sample is determined from the amount of the complex detected in the above-mentioned step (2').

The amount (concentration) of the complex detected in the step (2') can be measured by correlating a signal of fluorescence intensity or absorbance and the like to a separately prepared calibration curve. The concentration of HSP47 in a biological sample of a test subject can be quantified by multiplying the concentration obtained from the calibration curve by the dilution rate.

The calibration curve is shown by an approximation formula plotting the measured values, wherein the horizontal axis (vertical axis) shows the concentration of the samples obtained by serially diluting the aforementioned standard HSP47 protein, and the vertical axis (horizontal axis) shows the signal (in any unit) of the antibody bound to HSP47 in a standard HSP47 protein. The calibration curve can be shown in an approximate straight line by logarithmic presentation of the both axes.

Next, the amount of HSP47 in a biological sample derived from a test subject, and the possibility of being affected with acute lung injury are correlated, and whether or not the test subject is affected with acute lung injury is determined based on the correlation. As shown in the below-mentioned Examples, as compared to healthy human and patients with chronic progressive interstitial pneumonia, patients with acute lung injury show a higher amount of HSP47 in a biological sample. The above-mentioned determination is made based on the positive correlation between such amount of HSP47 in a biological sample derived from a test subject and the possibility of being affected with acute lung injury.

For example, biological samples (e.g., serum) are collected from healthy human or patients with chronic progressive interstitial pneumonia (negative control), and patients with acute lung injury (positive control), the amount of HSP47 in the biological samples collected from the test subjects is compared with that of the positive control and negative control. Alternatively, a correlation diagram of the amount of HSP47 in a particular biological sample (e.g., serum) and acute lung injury morbidity rate is prepared in advance, and the amount of HSP47 in a biological sample collected from a test subject may be compared with the correlation diagram. The amount of HSP47 is preferably compared based on the presence or absence of a significant difference.

From the comparison results of the amount of HSP47, when the amount of HSP47 in a biological sample derived from a test subject is relatively high, the test subject can be determined to have a relatively high possibility of being affected with acute lung injury. Conversely, when the amount of HSP47 in a biological sample derived from a test subject is relatively low, the test subject can be determined to have a relatively low possibility of being affected with acute lung injury. In a differentiation of acute lung injury from chronic progressive interstitial pneumonia using the method (2) of the present invention, when the amount of HSP47 in a biological sample derived from a test subject is relatively high, the test subject can be determined to have a relatively high possibility of being affected with acute lung injury rather than chronic progressive interstitial pneumonia. Conversely, when the amount of HSP47 in a biological sample derived from a test subject is relatively low, the test subject can be determined to have a high possibility of being affected with chronic progressive interstitial pneumonia rather than acute lung injury.

In a further aspect, acute lung injury can be distinguished from the above-mentioned diseases showing clinical findings common with acute lung injury by using the method (2) of the present invention. Clinical findings of acute lung injury are similar to those of the above-mentioned diseases. Since the above-mentioned diseases showing clinical findings common with acute lung injury do not have a factor that promotes collagen synthesis, the amount of HSP47 in the biological sample does not increase.

Specifically, for example, biological samples (e.g., serum) are collected from healthy human or patients with the above-mentioned diseases showing clinical findings common with acute lung injury (negative control), and patients with acute lung injury (positive control), the amount of HSP47 in the biological samples collected from the test subjects is compared with that of the positive control and negative control. Alternatively, a correlation diagram between the amount of HSP47 in a particular biological sample (e.g., serum) and acute lung injury morbidity rate is prepared in advance, and the amount of HSP47 in a biological sample collected from a test subject may be compared with the correlation diagram. The amount of HSP47 is preferably compared based on the presence or absence of a significant difference.

When the amount of HSP47 in a biological sample derived from a test subject is relatively high, the test subject can be determined to have a relatively high possibility of being affected with acute lung injury rather than the above-mentioned diseases showing clinical findings common with acute lung injury. Conversely, when the amount of HSP47 in a biological sample derived from a test subject is relatively low, the test subject can be determined to have a high possibility of being affected with the above-mentioned diseases showing clinical findings common with acute lung injury rather than acute lung injury.

Moreover, the present invention provides use of a diagnostic reagent for acute lung injury comprising the aforementioned collagen or a partial fragment thereof having specific affinity for HSP47. Said diagnostic reagent can be a diagnosis kit for acute lung injury. Using the diagnostic reagent of the present invention, whether or not a test subject is affected with acute lung injury can be easily determined by quantifying HSP47 in a biological sample derived from a test subject by an immunological means in the aforementioned method (2) of the present invention, or acute lung injury can be distinguished from chronic progressive interstitial pneumonia or the above-mentioned diseases showing clinical findings common with acute lung injury.

The collagen or a partial fragment thereof having specific affinity for HSP47 can also be used in unbound state in an aqueous solution, and it is preferably bound to a solid phase. Examples of the "solid phase" include a plate (e.g., a microwell plate), a tube, beads (e.g., plastic beads, magnetic beads), a chromatography carrier (e.g., Sepharose (trade mark)), a membrane (e.g., a nitrocellulose membrane, a PVDF membrane), a gel (e.g., a polyacrylamide gel), a metal film (e.g., a gold film) and the like.

The diagnostic reagent may comprise, besides collagen or a partial fragment thereof having specific affinity for HSP47, a reagent to be used for quantifying the amount of HSP47 in a biological sample and the like. These reagents may be stored in advance together with the collagen or a partial fragment thereof having specific affinity for HSP47, or stored in separate containers. Examples of the reagent include a treatment liquid, a buffer for diluting an antibody, an antibody that specifically recognizes HSP47 derived from a test subject, a labeling substance (e.g., a fluorescence dye, an enzyme), a reaction vessel, a positive control (e.g., a recombinant HSP47), a negative control, a solid phase, an instruction sheet describing the examination protocol and the like. These components can also be mixed in advance as necessary.

### (2) Use of heat shock protein 47 as a biomarker for predicting the onset of acute lung injury, in hyperacute stage before meeting the clinical definition of acute lung injury

In another embodiment of the present invention, the onset of acute lung injury can be predicted in hyperacute stage before meeting the clinical definition of acute lung injury, by quantifying heat shock protein 47 in a biological sample derived from a test subject.

In the embodiment, the "test subject" can be a test subject who fails to meet the clinical definition of acute lung injury. Examples of the test subject include, but are not limited to, healthy human, a test subject affected with interstitial pneumonia, and a test subject affected with an acute respiratory disease such as bacterial pneumonia, aspiration pneumonia and the like. The test subject can be a test subject showing no clinical finding of acute lung injury.

The heat shock protein 47 in the above-mentioned biological sample derived from a test subject can be quantified in the same manner as in the above-mentioned method (1) of the present invention and the method (2) of the present invention.

The amount of HSP47 in the above-mentioned biological sample derived from a test subject is correlated with the possibility of developing acute lung injury, and whether or not the test subject will develop acute lung injury is determined based on the correlation. In the above-mentioned test subjects, the higher the amount of HSP47 in the biological sample, the higher the possibility of developing acute lung injury. The above-mentioned determination is made based on the positive correlation between such amount of HSP47 in a biological sample derived from a test subject and the possibility of developing acute lung injury.

For the above-mentioned determination, for example, a correlation diagram of the amount of HSP47 in a particular biological sample (e.g., serum) and the possibility of developing acute lung injury may be prepared in advance, and the amount of HSP47 in a biological sample collected from a test subject may be compared with the correlation diagram.

From the comparison results of the amount of HSP47, when the amount of HSP47 in a biological sample derived from a test subject is relatively high, the test subject can be determined to have a high possibility of developing acute lung injury. Conversely, when the amount of HSP47 in a biological sample derived from a test subject is relatively low, the test subject can be determined to have a low possibility of developing acute lung injury.

When serum HSP47 increases during the follow-up of patients with interstitial pneumonia, high possibility of acute exacerbation in a short span and the onset of rapidly progressive interstitial pneumonia meeting the clinical definition of acute lung injury can be predicted. Since acute exacerbation of interstitial pneumonia shows only the general common cold-like symptoms in the early stages of the onset, it is often treated as a simple cold, during which the severity increases and often becomes fatal. Even when acute exacerbation of interstitial pneumonia is suspected, an early diagnosis is difficult since hypoxia or abnormal shadow in chest radiography is found only after exacerbation reaches a certain level. However, even when a patient with interstitial pneumonia shows extremely mild symptoms, the measurement of serum HSP47 enables determination of a low possibility of developing acute exacerbation when the measured value is low or a high possibility of detecting a very early stage of acute exacerbation when the measured value is high. Thus, the very early stage of interstitial pneumonia patients developing acute exacerbation can be diagnosed, and the onset of acute exacerbation can be predicted even before the onset thereof.

Similarly, also in acute respiratory diseases such as bacterial pneumonia, aspiration pneumonia and the like, the measurement of serum HSP47 enables determination of a low possibility of acute exacerbation to develop acute lung injury when the measured value is low, or a high possibility of detecting a very early stage of acute exacerbation developing acute lung injury when the measured value is high.

### (3) Comprehension of the activity of acute lung injury, and use of heat shock protein 47 as a biomarker for predicting prognosis

In another embodiment of the present invention, activity of acute lung injury can be comprehended and prognosis of acute lung injury can be predicted by quantifying heat shock protein 47 in a biological sample derived from a test subject affected with acute lung injury.

Since the HSP47 value in the serum of a test subject affected with acute lung injury is correlated with the disease activity of acute lung injury, the activity and severity can be comprehended, the treatment effect can be evaluated, and the prognosis can be predicted by measuring HSP47 in the serum.

The heat shock protein 47 in a biological sample derived from a test subject affected with acute lung injury can be quantified in the same manner as in the above-mentioned method (1) of the present invention and the method (2) of the present invention.

The amount of HSP47 in a biological sample derived from a test subject affected with acute lung injury is correlated with the disease activity of acute lung injury, and the activity and severity of acute lung injury in the test subject is determined based on the correlation. In this determination, for example, a correlation diagram of the amount of HSP47 in a particular biological sample (e.g., serum) and disease activity of acute lung injury is prepared in advance, and the amount of HSP47 in the biological sample collected from a test subject is compared with the correlation diagram.

### Examples

The present invention is explained in more detail in the following by referring to Examples, which are not to be construed as limitative.

### Test subjects

The test subjects were patients at Nagasaki University Hospital, including ALI (32 cases), COP (12 cases), idiopathic UIP (I-UIP; 19 cases), idiopathic NSIP (I-NSIP; 16 cases), CVD-UIP (11 cases), CVD-NSIP (12 cases) and voluntary healthy subjects (18 cases). The ALI cases include rapidly progressive interstitial pneumonia meeting the clinical definition of ALI. The test protocol was approved by the institutional review board, and informed consent was obtained from the test subjects. Further specific clinical backgrounds are shown in Table 1. In the Table, P/F ratio, A-aDO₂ and Respiratory index show PaO_{2/}FiO₂ (mmHg) (arterial partial pressure of oxygen divided by fraction of inspired oxygen), alveolar-arterial oxygen difference (difference between alveolar partial pressure of oxygen (PAO₂) and arterial partial pressure of oxygen (PaO₂)) and A-aDO₂/PaO₂ (alveolar-arterial oxygen difference divided by arterial partial pressure of oxygen), respectively, and KL-6, SP-D, SP-A and LDH show serum marker values. P/F ratio, A-aDO₂ and respiratory index showed a statistically significant difference at p<0.01 by comparison of ALI patients with patients with COP, idiopathic UIP, idiopathic NSIP, CVD-UIP and CVD-NSIP.

**Table 1**

| Median,(Range) | | | | | | | | | | | | | | | |
|---|---|---|---|---|---|---|---|---|---|---|---|---|---|---|---|
| | healthy (control) | | COP | | I-UIP | | I-NSIP | | CVD-UIP | | CVD-NSIP | | ALI (ex19-4-5) | | Kruskal-Wallis Test p value |
| | | | | | | | | | | | | | | | |
| Age (years) | 36.5 | 27-59 | 65.5 | 38-87 | 64.0 | 34-75 | 57.0 | 28-74 | 60.0 | 41-71 | 59.5 | 40-71 | 69.0 | 16-82 | <0.0001 |
| Sex (male/ female) | 11/7 | | 7/5 | | 13/6 | | 6/10 | | 3/8 | | 3/9 | | 17/15 | | N.S. (x-square test) |
| | | | | | | | | | | | | | | | |
| P/F ratio | | | 385.0 | 289.5-490.5 | 389.8 | 283.3-491.9 | 399.3 | 343.3-442.4 | 393.3 | 339.5-471.9 | 382.9 | 330.5-443.8 | 129.6 | 55.4-282.4 | <0.0001 |
| A-a DO2 | | | 19.9 | -5.14-46.9 | 17.1 | -3.5-52.7 | 15.3 | 2.6-37.9 | 16.5 | -8.9-36.9 | 35.1 | 0.03-48.5 | 307.3 | 38.8-611.4 | <0.0001 |
| Respiratory index | | | 0.24 | -0.05-0.77 | 0.21 | -0.03-0.77 | 0.18 | 0.03-0.53 | 0.20 | -0.09-0.52 | 0.43 | 0.0003-0.7 | 3.79 | 0.65-10.9 | <0.0001 |
| | | | | | | | | | | | | | | | |
| KL-6 | 195 | 144-322 | 427.5 | 172-1310 | 1460.0 | 444-4340 | 1568.5 | 192-4745 | 786.0 | 348-1610 | 848.0 | 149-2550 | 757.0 | 158-5800 | <0.0001 |
| SP-D | 17.3 | 17.3-53.3 | 105.7 | 27.8-247 | 316.0 | 93.1-721 | 500.0 | 49.2-942 | 187.0 | 33.5-264 | 116.0 | 33.2-275 | 223.0 | 29-4510 | <0.0001 |
| SP-A | 22.8 | 12.1-60.8 | 52.8 | 20.6-129 | 103.0 | 62.4-355 | 48.9 | 20.3-127 | 92.9 | 49.6-114 | 47.6 | 26.2-253 | 112.0 | 43.8-328 | <0.0001 |
| LDH | 124.5 | 20-246 | 164.0 | 132-236 | 233.0 | 113-416 | 212.5 | 135-738 | 373.0 | 172-474 | 243.5 | 122-670 | 380.0 | 177-2845 | <0.0001 |

### [Example 1]

### Preparation of HSP47 protein

### Preparation of collagen-immobilized column

CNBr-activated Sepharose 4B (GE Bioscience) was suspended in 1 mM HCl, and the suspension was mixed with the equal amount of 1 mM HCl solution of 3 mg/ml swine type I collagen (Nitta Gelatin Inc.). A 2-fold amount of 0.2 M NaHCO₃ and 0.5 M NaCl were added, and the mixture was stirred using a rotator at room temperature for 2 hr. The resin was recovered by centrifugation, suspended in 1 M ethanolamine hydrochloride (pH 8.5), and the suspension was stirred at 4°C for 1 hr. The resin was washed with 0.2 M NaHCO₃ 0.5 M NaCl, and then with 0.1 M sodium acetate buffer (pH 4.0) containing 0.5 M sodium chloride. This was used as a collagen-Sepharose 4B resin for HSP47 purification.

### Preparation of recombinant HSP47 protein

cDNA of human HSP47 was subcloned to pET3a to construct an expression plasmid (pET-hH47). *Escherichia coli* BL21 (DE3) was transformed with pET-hH47. The transformants were cultured at 25°C, 0.1 mM isopropyl thiogalactoside was added, and they were further cultured for 2 hr. The following purification operation was performed at 4°C. Recombinant *Escherichia coli* cells were recovered by centrifugation, and suspended in an extraction buffer (50 mM Tris hydrochloride, pH 8.0, 150 mM sodium chloride, 5 mM EDTA, 1 mM phenylmethylsulfonyl fluoride, 1 µg/ml pepstatin A, 1 µg/ml leupeptin, 0.2% Nonidet P40, 30% glycerol). After ultrasonication, the suspension was centrifuged at 12,000 rpm for 15 min, the supernatant was applied to collagen-Sepharose 4B column equilibrated with 150 mM sodium chloride-containing 50 mM Tris-hydrochloride, pH 8.0 (bed volume 360 ml per 1 liter culture, flow rate 1 ml/min). The column was washed with 50 mM Tris-hydrochloride, pH 8.0, 150 mM sodium chloride and 1% Nonidet P40. HSP47 bound to the resin was eluted with a solution of MES buffer, pH 5.8, 150 mM NaCl, 1% Nonidet P40 and 10% glycerol. The eluate was neutralized with 1/10 amount of 1 M Tris hydrochloride, pH 8.0, and glycerol was added to a final concentration of 20%.

### [Example 2]

### Production of antibody immobilized plate

Fifty µL/well of rabbit anti-HSP47 polyclonal antibody (manufactured by Santa Cruz Biotechnology) diluted with 50 mM sodium carbonate buffer (pH 9.6) to 1.0 µg/mL was added to a 96-well microplate (manufactured by Nunc), and the plate was left standing at 4°C overnight to bind the antibody to the plate surface. After discarding the antigen solution, 200 µL/well of a phosphate buffer containing 2% BSA (manufactured by Sigma Ltd.) was added to suppress non-specific adsorption to the plate and non-specific reaction, and the plate was left standing at room temperature for 2 hr to carry out blocking the plate. Then, the well was washed three times with PBS containing 0.05% Tween 80 (PBST) to prepare an anti-HSP47 antibody immobilized plate.

### Detection of recombinant HSP47 and preparation of calibration curve

As recombinant HSP47, a product purified by the above-mentioned method was used. To the plate prepared as above was added 100 µL/well of the aforementioned recombinant HSP47 solution (in 1% BSA-PBST, pH 7.4) with various concentrations, and the plate was left standing at room temperature for 2 hr to form a complex of anti-HSP47 antibody bound to recombinant HSP47. The well was washed three times with PBST, and mouse anti-HSP47 monoclonal antibody (0.5 µg/mL, manufactured by Stressgen) was added at 100 µL/well. The plate was left standing at 37°C for 2 hr to bind the mouse anti-HSP47 monoclonal antibody to the recombinant HSP47 in the complex. The well was washed three times with PBST, POD-labeled antimouse IgG antibody (diluted 10,000-fold, manufactured by Biosource) was added at 100 µL/well, and the plate was left standing at 37°C for 2 hr. The well was washed three times with PBST, 100 µL/well of 3,3',5,5'-tetramethylbenzidine substrate solution (manufactured by Biosource) was added, and the plate was left standing at room temperature for 15 min to allow color development. The reaction was quenched with the equal amount of 1M phosphoric acid, and the absorbance at 450 nm was measured. A calibration curve was prepared based on the obtained data.

### [Example 3]

### Detection of HSP47 by using human serum as a sample

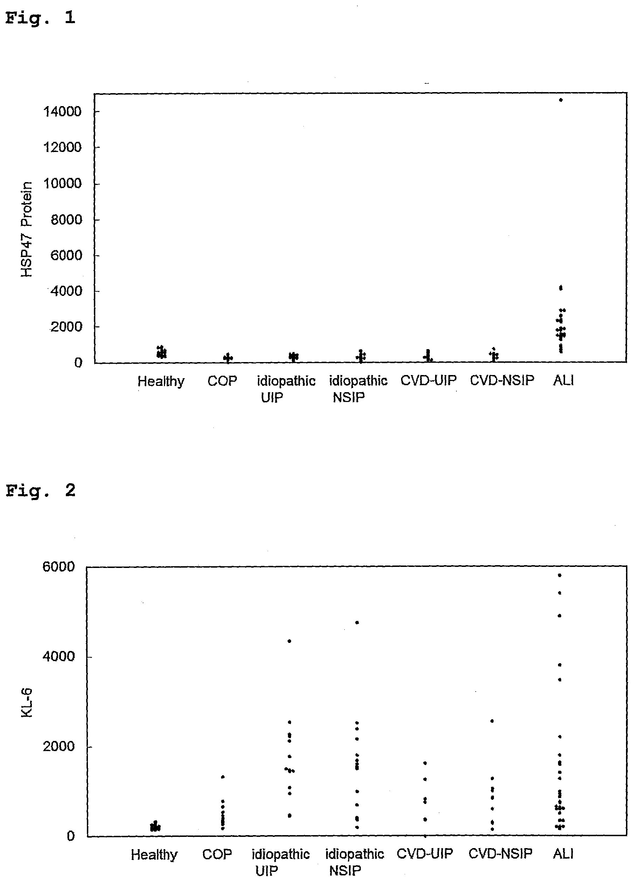

By an operation similar to that in Example 2 except that the serum of a patient affected with acute lung injury or various kinds of chronic progressive interstitial pneumonia was used as a sample instead of the recombinant HSP47 solution, the absorbance was measured. Based on the calibration curve prepared in Example 2, the concentration of HSP47 in the serum of each patient was calculated. The results are shown in Fig. 1.

While the HSP47 concentrations of the patients with chronic progressive interstitial pneumonia (COP, I-UIP, I-NSIP, CVD-UIP, CVD-NSIP) and healthy humans were of a low level, patients with acute lung injury showed an extremely high concentration level (Fig. 1). When the HSP47 concentration was respectively compared between ALI patients and patients with COP, I-UIP, I-NSIP, CVD-UIP and CVD-NSIP or healthy humans, a statistically significant difference was found at p<0.05. Therefore, whether or not a patient is affected with acute lung injury can be determined using the blood HSP47 concentration as an index.

### [Comparative Example 1]

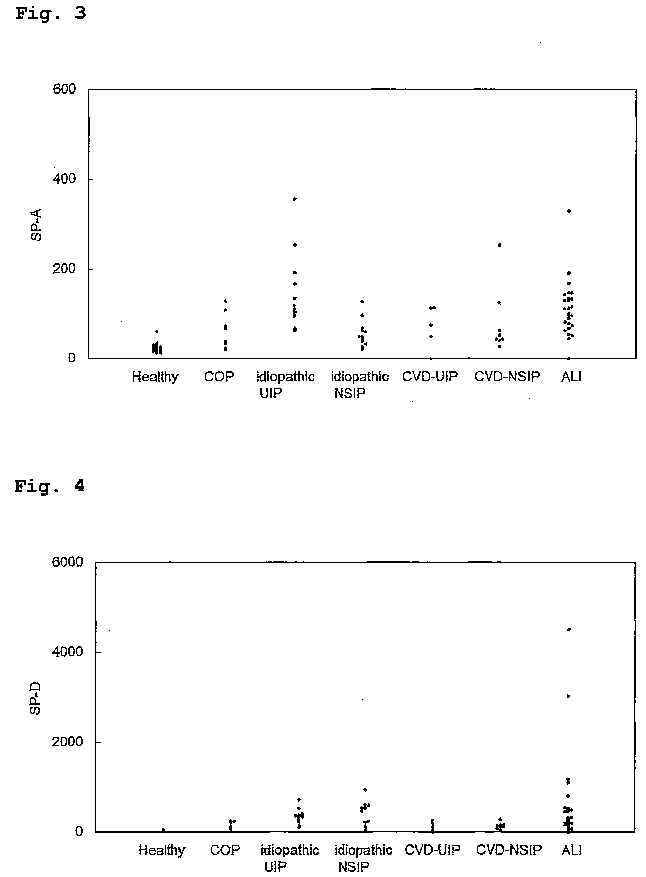

The measurement was also performed for KL-6, SP-D, SP-A and LDH by using the sera of the above-mentioned test subjects. Respective results are shown in Figs. 2-5. No significant difference was found for any serum markers between patients with chronic progressive interstitial pneumonia and acute lung injury.

### [Example 4]

### Receiver Operating Characteristic (ROC) curve analysis

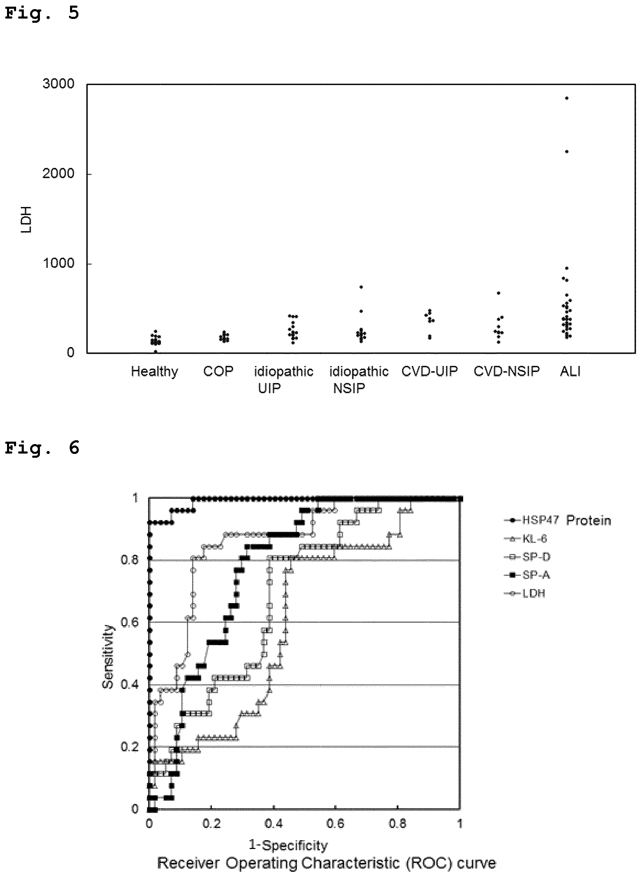

Receiver operating characteristic (ROC) curves were drawn based on the measurement values of the respective serum markers of HSP47 protein, KL-6, SP-D, SP-A and LDH in the test subjects (see Shuji Matsuo and Hiroshi Takahashi: Practical Application of Receiver Operating Characteristic Curve on Laboratory Diagnosis. Rinsho Byori 42:585-590, 1994 as to the method for preparation). The results are shown in Fig. 6.

The ROC curve of the HSP47 protein value in the serum was distributed at a position nearest to the upper left corner where the acute lung injury disease group can be completely separated from the chronic progressive interstitial pneumonia disease group, as compared to the ROC curves of KL-6 value, SP-D value, SP-A value and LDH value.

Furthermore, by setting the cutoff value of the HASP47 protein in the serum to 859.3 pg/mL, acute lung injury and chronic progressive interstitial pneumonia could be distinguished with sensitivity 92.3% and specificity 98.2%. Therefore, the results show that HSP47 is a marker far more useful than the conventional serum markers KL-6, SP-D, SP-A and LDH.

In this study, the death rate after 1 month was 31.3% (10 cases/32 cases) and that after 3 months was 50% (16 cases/32 cases) for ALI. However, in chronic progressive interstitial pneumonia (COP, I-UIP, I-NSIP, CVD-UIP, CVD-NSIP), all patients were alive after 1 month and 3 months. The results show that HSP47 is a marker useful as a prognosis prediction factor for patients.

### [Example 5]

The tests were performed by further increasing the number of the target test subjects.

### Population subjected to investigation for HSP47 in serum

The test subjects of this study consisted of 116 patients admitted to Nagasaki University Hospital from April 1991 to April 2011 and 18 adult healthy human volunteers. The patients included 47 patients with rapidly progressive interstitial pneumonia (RPIP) included in acute lung injury in the present invention, 12 patients with cryptogenic organizing pneumonia (COP), 19 patients with idiopathic UIP, 16 patients with idiopathic NSIP, 11 patients with CVD-UIP, and 11 patients with CVD-NSIP. Patients meeting the following four criteria: 1) exacerbated respiratory condition by aggressive progression within 30 days; 2) detection of the first bilateral ground glass opacity and/or infiltration shadow by high resolution chest CT scan; 3) resting PaO₂/fraction of inspired oxygen (FiO₂) ratio (P/F ratio) <300 mmHg; and 4) lack of clear infection, pneumothorax, pulmonary thromboembolism, heart failure or other cause of acute lung injury (physical trauma, blood transfusion or toxic inhaled toxicant) were diagnosed as RPIP. The patients with RPIP consisted of 17 patients with acute exacerbation of IPF, 10 patients with acute interstitial pneumonia (AIP), 8 patients with collagen vascular disease-associated interstitial pneumonia and 12 patients with drug-induced interstitial pneumonia.

### Statistical analysis

The values of continuous variables were expressed as median (range). Differences among groups were determined by analysis of variance or the Kruscal-Wallis test for continuous variables and the χ² test for categorical variables, as appropriate. If a significant difference was found by analysis of variance, pair-wise comparison was performed using the Scheffe method. The upper left corner coordinate point of the receiver operating characteristic curve was used to determine the optimum cutoff level for discriminating between RPIP and other interstitial pneumonias (COP, idiopathic UIP, idiopathic NSIP, CVD-UIP and CVD-NSIP). The statistical analysis was performed using a statistical software package (SAS 9.1.3, SAS Institute, Cary, NC). p value<0.05 was considered statistically significant.

### Characteristics of patients

The characteristics of the patients collected for the study are shown in Table 2. The P/F ratios of the RPIP group were significantly lower (P<0.01) as compared with those of the COP, idiopathic UIP, idiopathic NSIP, CVD-UIP and CVD-NSIP groups. In the RPIP group, alveolar-arterial difference of oxygen (A-a DO₂) was significantly high (P<0.01) as compared to the COP, idiopathic UIP, idiopathic NSIP, CVD-UIP and CVD-NSIP groups. The APACHE (Acute Physiology and Chronic Health Evaluation) II score and SOFA (Sequential Organ Failure Assessment) score in the RPIP group were 15.0 (range 8 - 35) and 4.0 (range 2 - 12), respectively.

**Table 2**

| Median (range) | | | | | | | | | | | | | |
|---|---|---|---|---|---|---|---|---|---|---|---|---|---|
| | COP | | I-UIP | | I-NSIP | | CVD-UIP | | CVD-NSIP | | RPIP | | p value |
| | | | | | | | | | | | | | |
| Age (years) | 65.5 | (38-87) | 64.0 | (34-75) | 57.0 | (28-74) | 60.0 | (41-71) | 58.0 | (40-71) | 69.0 | (16-82) | <0.05 |
| Sex (male/female) | 7/5 | | 13/6 | | 6/10 | | 3/8 | | 2/9 | | 30/17 | | <0.05 |
| | | | | | | | | | | | | | |
| P/F ratio | 385.0 | (289.5-490.5) | 389.8 | (283.3-491.9) | 399.3 | (343.3-442.4) | 393.3 | (339.5-471.9) | 378.6 | (330.5-443.8) | 124.7 * | (53-287.1) | <0.0001 |
| A-a DO2 | 19.9 | (-5.14-46.9) | 17.1 | (-3.5-52.7) | 15.3 | (2.6-37.9) | 16.5 | (-8.9-36.9) | 35.4 | (0.03-48.5) | 363.2 * | (38.7-611.4) | <0.0001 |

| | | | | | | | | | | | | | |
|---|---|---|---|---|---|---|---|---|---|---|---|---|---|
| Data shows median value (range). COP= cryptogenic organizing pneumonia; UIP= usual interstitial pneumonia; NSIP= nonspecific interstitial pneumonia; P/F ratio = resting PaO₂/fraction of inspired oxygen (FiO₂) ratio; A-a DO₂ = alveolar-arterial oxygen difference * p>0.01 compared to COP, idiopathic UIP, idiopathic NSIP, CVD-UIP and CVD-NSIP | | | | | | | | | | | | | |

### Outcome of disease

In the RPIP group, the 30-day mortality rate was 34.0% (16 out of 47 patients) and the 90-day mortality rate was 51.1% (24 out of 47 patients). In contrast, no patient died within 90 days in the COP, idiopathic UIP, idiopathic NSIP, CVD-UIP and CVD-NSIP groups.

### Levels of HSP47, KL-6, SP-A, SP-D and LDH in serum

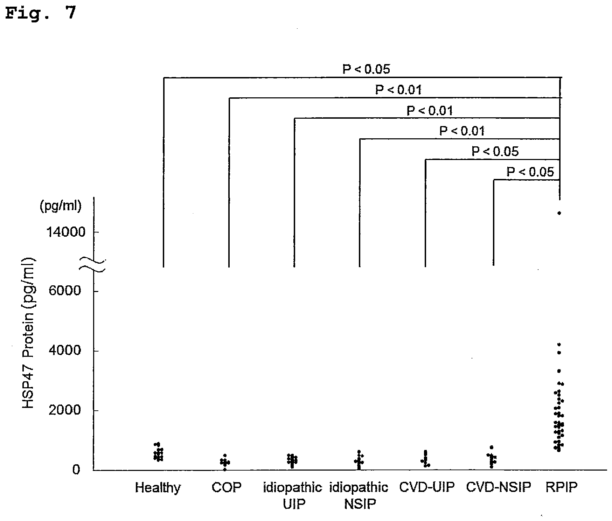

The serum HSP47 levels of the patients were quantified in the same manner as in Example 2. The serum HSP47 levels of the RPIP patients (median value 1530..2 [range 631.2 - 14589.9] pg/ml) were significantly higher than those of COP (239.1 [16.6 - 476.6] pg/ml) (P<0.01), idiopathic UIP (330.9 [105.1 - 487.6] pg/ml) (P<0.01), idiopathic NSIP (290.7 [24.8 - 603.0] pg/ml) (P<0.01), CVD-UIP (348.8 [122.8 - 602.0] pg/ml) (P<0.05), CVD-NSIP (383.1 [101.9 - 721.4] pg/ml) (P<0.05), and healthy volunteers (547.9 [332.1 - 879.8] pg/ml) (P<0.05). Serum levels of HSP47 among patients with COP, idiopathic UIP, idiopathic NSIP, CVD-UIP and CVD-NSIP, and healthy volunteers were not significantly different (Fig. 7).

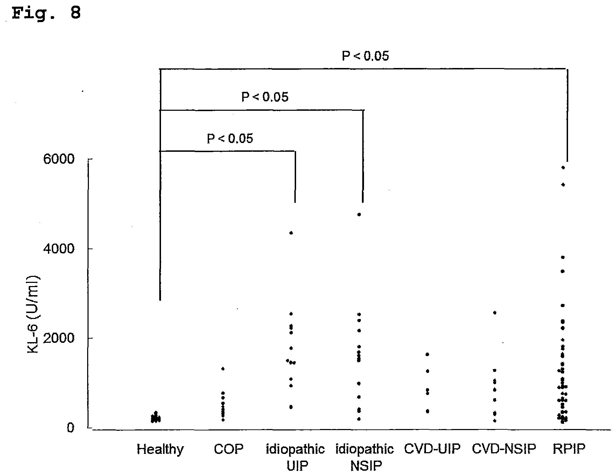

The serum KL-6 levels of the patients with idiopathic UIP, idiopathic NSIP and RPIP (idiopathic UIP patients (1460.0 [444 - 4340] U/ml) (P<0.05), idiopathic NSIP patients (1568.5 [192 - 4745] U/ml) (P<0.05) and RPIP patients (918.5 [107 - 5800] U/ml) (P<0.05)) were significantly higher than those of healthy volunteers (195.0 [144 - 322] U/ml). However, the serum KL-6 levels of the patients with RPIP showed no significant difference as compared to those of the patients with each of COP (427.5 [172 - 1310] U/ml), idiopathic UIP, idiopathic NSIP, CVD-UIP (786.0 [348 - 1610] U/ml) and CVD-NSIP (924.0 [149 - 2550] U/ml) (Fig. 8).

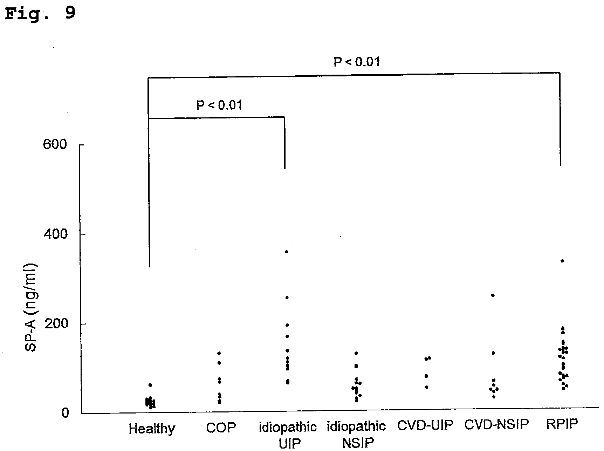

The serum SP-A levels of the patients with RPIP and idiopathic UIP (RPIP patients (123.0 [43.0 - 328.0] ng/ml) (P<0.01) and idiopathic UIP patients (103.0 [62.4 - 355.0] ng/ml) (P<0.01)) were significantly higher than those of healthy volunteers (22.8 [12.1 - 60.8] ng/ml). However, the serum SP-A levels of the RPIP patients showed no significant difference as compared to each of the patients with COP (52.8 [20.6 - 129.0] ng/ml), idiopathic UIP, idiopathic NSIP (48.9 [20.3 - 127.0] ng/ml), CVD-UIP (92.9 [49.6 - 114.0] ng/ml), and CVD-NSIP (51.9 [26.2 - 253.0] ng/ml) (Fig. 9).

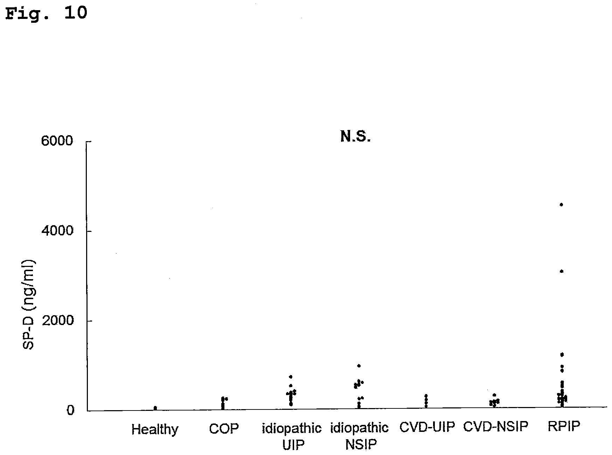

The patients with COP, idiopathic UIP, idiopathic NSIP, CVD-UIP, CVD-NSIP and RPIP (COP patients (105.7 [27.8 - 247.0] ng/ml), idiopathic UIP patients (316.0 [93.1 - 721.0] ng/ml), idiopathic NSIP patients (477.0 [17.2 - 942.0] ng/ml), CVD-UIP patients (187.0 [33.5 - 264.0] ng/ml), CVD-NSIP patients (111.5 [33.2 - 275.0] ng/ml), RPIP patients (280.5 [29.0 - 4510.0] ng/ml)), and healthy volunteers (17.3 [17.3 - 53.3] ng/ml) showed no significant difference in the serum SP-D levels (Fig. 10).

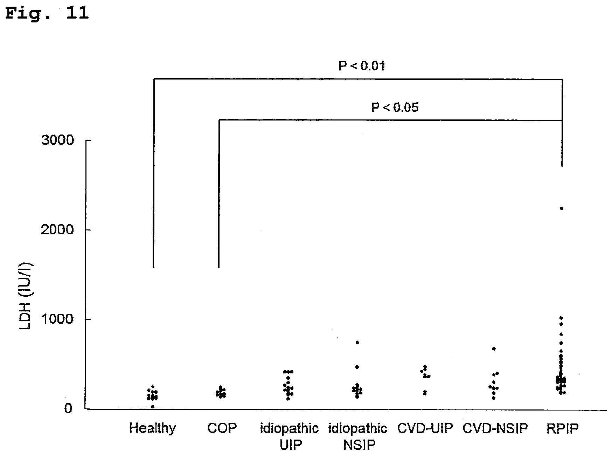

The serum LDH levels of the RPIP patients (368.0 [177 - 2250] IU/l) was significantly higher than those of the COP patients (164.0 [132 - 236] IU/l) (P<0.05) and healthy volunteers (124.5 [20 - 246] IU/l) (P<0.01). However, the serum LDH levels of the RPIP patients showed no significant difference as compared to each of the patients with idiopathic UIP (233.0 [113 - 416] IU/l), idiopathic NSIP (212.5 [135 - 738] IU/l), CVD-UIP (373.0 [172 - 474] IU/l), and CVD-NSIP (247.0 [181 - 670] IU/l) (Fig. 11).

### [Example 6]

### Receiver Operating Characteristic curve

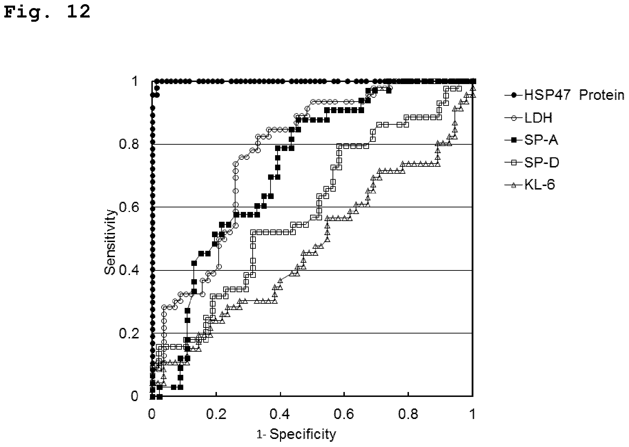

Receiver operating characteristic curves were drawn in the same manner as in Example 4 (Fig. 12). Based on the receiver operating characteristic curves, the HSP47 cutoff value enabling diagnosis with highest accuracy was 617.1 pg/ml. The HSP47 cutoff value of 617.1 pg/ml distinguished RPIP and other interstitial pneumonias (COP, idiopathic UIP, idiopathic NSIP, CVD-UIP, and CVD-NSIP) with 100% sensitivity and 98.6% specificity. The diagnostic accuracy was 99.1%. Use of serum HSP47 levels resulted in the largest area under the curve. Using the serum HSP47 level, the area under the curve in the diagnosis of rapidly progressive interstitial pneumonia was 0.998. In contrast, the areas under the curve in the diagnosis of rapidly progressive interstitial pneumonia based on the serum KL-6, SP-A, SP-D and LDH levels were only 0.475, 0.728, 0.595 and 0.776, respectively.

The results show that HSP47 is a marker far more useful than conventional serum markers KL-6, SP-D, SP-A and LDH.

### Industrial Applicability

According to the examination method of the present invention, a novel biomarker HSP47 for acute lung injury can be detected easily and rapidly. In addition, using the kit for diagnosis of acute lung injury and the diagnostic reagent for acute lung injury of the present invention, whether or not a patient is affected with acute lung injury can be diagnosed conveniently, rapidly and highly accurately.

When a patient with symptoms of cough, respiratory distress, fever and the like and a diffuse shadow in chest radiography receives a medical examination in an actual clinical practice, it is often difficult to predict whether the patient thereafter shows chronic progression, or rapid progression. Measurement of HSP47 enables a convenient, aggressive and highly accurate diagnosis of whether the patient is affected with acute lung injury that will show rapid and lethal progression or chronic progressive interstitial pneumonia that will show chronic progression.

This application is based on a patent application No. 2011-027679 filed February 10, 2011 in Japan.

## Claims

1. A method of determining whether or not a test subject is affected with acute lung injury, comprising quantifying heat shock protein 47 in a biological sample derived from a test subject.

2. The method according to claim 1, which distinguishes acute lung injury from chronic progressive interstitial pneumonia.

3. The method according to claim 1 or 2, wherein the biological sample is blood, serum or plasma.

4. Use of a reagent comprising an antibody that specifically recognizes heat shock protein 47 and/or collagen or a partial fragment thereof that has specific affinity for heat shock protein 47 for diagnosing acute lung injury *ex vivo.*

5. A method of evaluating a treatment effect for or predicting the prognosis of acute lung injury of a test subject, comprising quantifying heat shock protein 47 in a biological sample derived from the test subject.

## Patentansprüche

1. Verfahren zur Bestimmung, ob ein Proband an akuter Lungenschädigung leidet oder nicht, umfassend das Quantifizieren von Hitzeschockprotein 47 in einer von dem Probanden stammenden biologischen Probe.

2. Verfahren gemäß Anspruch 1, das eine akute Lungenschädigung von einer chronischen progressiven interstitiellen Pneumonie unterscheiden kann.

3. Verfahren gemäß Anspruch 1 oder 2, wobei es sich bei der biologischen Probe um Blut, Serum oder Plasma handelt.

4. Verwendung eines Reagens, umfassend einen Antikörper, der spezifisch das Hitzeschockprotein 47 erkennt, und/oder Collagen oder ein Fragment davon, das eine spezifische Affinität zu Hitzeschockprotein 47 aufweist, zum Diagnostizieren einer akuten Lungenschädigung ex vivo.

5. Verfahren zur Bewertung einer Behandlungswirkung bezüglich einer akuten Lungenschädigung oder zur Prognose einer akuten Lungenschädigung eines Probanden, umfassend das Quantifizieren von Hitzeschockprotein 47 in einer von dem Probanden stammenden biologischen Probe.

## Revendications

1. Méthode pour déterminer si un sujet sous test est affecté ou non par une lésion pulmonaire aiguë, comprenant la quantification de la protéine de choc thermique 47 dans un échantillon biologique dérivé d'un sujet sous test.

2. Méthode selon la revendication 1, laquelle distingue une lésion pulmonaire aiguë d'une pneumonie interstitielle progressive chronique.

3. Méthode selon la revendication 1 ou 2, dans laquelle l'échantillon biologique est du sang, du sérum ou du plasma.

4. Utilisation d'un réactif comprenant un anticorps qui reconnaît de manière spécifique la protéine de choc thermique 47 et/ou un collagène ou un fragment partiel de celui-ci qui présente une affinité spécifique pour la protéine de choc thermique 47 pour diagnostiquer une lésion pulmonaire aiguë *ex vivo.*

5. Méthode d'évaluation de l'effet d'un traitement ou de prédiction du pronostic pour une lésion pulmonaire aiguë d'un sujet sous test, comprenant la quantification d'une protéine de choc thermique 47 dans un échantillon biologique dérivé d'un sujet sous test.


### SEQUENCE LISTING

<110> NAGASAKI UNIVERSITY SAPPORO MEDICAL UNIVERSITY
<120> Method of Diagnosing Acute Lung Injury
<130> 091795
<150> JP 2011-027679
   <151> 2011-02-10
<160> 6
<170> PatentIn version 3.1
<210> 1
   <211> 2208
   <212> DNA
   <213> Homo sapiens
<220>
   <221> CDS
   <222> (230)..(1486)
   <223>
<400> 1
   tctttggctt tttttggcgg agctggggcg ccctccggaa gcgtttccaa ctttccagaa     60
   gtttctcggg acgggcagga gggggtgggg actgccatat atagatcccg ggagcagggg    120
   agcgggctaa gagtagaatc gtgtcgcggc tcgagagcga gagtcacgtc ccggcgctag    180
   cccagcccga cccaggccca ccgtggtgca cgcaaaccac ttcctggcc atg cgc tcc    238
                                                         Met Arg Ser
                                                         1
   ctc ctg ctt ctc agc gcc ttc tgc ctc ctg gag gcg gcc ctg gcc gcc      286
   Leu Leu Leu Leu Ser Ala Phe Cys Leu Leu Glu Ala Ala Leu Ala Ala
       5                   10                  15
   gag gtg aag aaa cct gca gcc gca gca gct cct ggc act gcg gag aag      334
   Glu Val Lys Lys Pro Ala Ala Ala Ala Ala Pro Gly Thr Ala Glu Lys
   20                  25                  30                  35
   ttg agc ccc aag gcg gcc acg ctt gcc gag cgc agc gcc ggc ctg gcc      382
   Leu Ser Pro Lys Ala Ala Thr Leu Ala Glu Arg Ser Ala Gly Leu Ala
                   40                  45                  50
   ttc agc ttg tac cag gcc atg gcc aag gac cag gca gtg gag aac atc      430
   Phe Ser Leu Tyr Gln Ala Met Ala Lys Asp Gln Ala Val Glu Asn Ile
               55                  60                  65
   ctg gtg tca ccc gtg gtg gtg gcc tcg tcg cta ggg ctc gtg tcg ctg      478
   Leu Val Ser Pro Val Val Val Ala Ser Ser Leu Gly Leu Val Ser Leu
           70                  75                  80
   ggc ggc aag gcg acc acg gcg tcg cag gcc aag gca gtg ctg agc gcc      526
   Gly Gly Lys Ala Thr Thr Ala Ser Gln Ala Lys Ala Val Leu Ser Ala
       85                  90                  95
   gag cag ctg cgc gac gag gag gtg cac gcc ggc ctg ggc gag ctg ctg      574
   Glu Gln Leu Arg Asp Glu Glu Val His Ala Gly Leu Gly Glu Leu Leu
   100                 105                 110                 115
   cgc tca ctc agc aac tcc acg gcg cgc aac gtg acc tgg aag ctg ggc        622
   Arg Ser Leu Ser Asn Ser Thr Ala Arg Asn Val Thr Trp Lys Leu Gly
                   120                 125                 130
   agc cga ctg tac gga ccc agc tca gtg agc ttc gct gat gac ttc gtg        670
   Ser Arg Leu Tyr Gly Pro Ser Ser Val Ser Phe Ala Asp Asp Phe Val
               135                 140                 145
   cgc agc agc aag cag cac tac aac tgc gag cac tcc aag atc aac ttc        718
   Arg Ser Ser Lys Gln His Tyr Asn Cys Glu His Ser Lys Ile Asn Phe
           150                 155                 160
   cgc gac aag cgc agc gcg ctg cag tcc atc aac gag tgg gcc gcg cag        766
   Arg Asp Lys Arg Ser Ala Leu Gln Ser Ile Asn Glu Trp Ala Ala Gln
       165                 170                 175
   acc acc gac ggc aag ctg ccc gag gtc acc aag gac gtg gag cgc acg        814
   Thr Thr Asp Gly Lys Leu Pro Glu Val Thr Lys Asp Val Glu Arg Thr
   180                 185                 190                 195
   gac ggc gcc ctg cta gtc aac gcc atg ttc ttc aag cca cac tgg gat        862
   Asp Gly Ala Leu Leu Val Asn Ala Met Phe Phe Lys Pro His Trp Asp
                   200                 205                 210
   gag aaa ttc cac cac aag atg gtg gac aac cgt ggc ttc atg gtg act        910
   Glu Lys Phe His His Lys Met Val Asp Asn Arg Gly Phe Met Val Thr
               215                 220                 225
   cgg tcc tat acc gtg ggt gtc atg atg atg cac cgg aca ggc ctc tac        958
   Arg Ser Tyr Thr Val Gly Val Met Met Met His Arg Thr Gly Leu Tyr
           230                 235                 240
   aac tac tac gac gac gag aag gaa aag ctg caa atc gtg gag atg ccc       1006
   Asn Tyr Tyr Asp Asp Glu Lys Glu Lys Leu Gln Ile Val Glu Met Pro
       245                 250                 255
   ctg gcc cac aag ctc tcc agc ctc atc atc ctc atg ccc cat cac gtg       1054
   Leu Ala His Lys Leu Ser Ser Leu Ile Ile Leu Met Pro His His Val
   260                 265                 270                 275
   gag cct ctc gag cgc ctt gaa aag ctg cta acc aaa gag cag ctg aag       1102
   Glu Pro Leu Glu Arg Leu Glu Lys Leu Leu Thr Lys Glu Gln Leu Lys
                   280                 285                 290
   atc tgg atg ggg aag atg cag aag aag gct gtt gcc atc tcc ttg ccc       1150
   Ile Trp Met Gly Lys Met Gln Lys Lys Ala Val Ala Ile Ser Leu Pro
               295                 300                 305
   aag ggt gtg gtg gag gtg acc cat gac ctg cag aaa cac ctg gct ggg       1198
   Lys Gly Val Val Glu Val Thr His Asp Leu Gln Lys His Leu Ala Gly
           310                 315                 320
   ctg ggc ctg act gag gcc att gac aag aac aag gcc gac ttg tca cgc       1246
   Leu Gly Leu Thr Glu Ala Ile Asp Lys Asn Lys Ala Asp Leu Ser Arg
       325                 330                 335
   atg tca ggc aag aag gac ctg tac ctg gcc agc gtg ttc cac gcc acc       1294
   Met Ser Gly Lys Lys Asp Leu Tyr Leu Ala Ser Val Phe His Ala Thr
   340                 345                 350                 355
   gcc ttt gag ttg gac aca gat ggc aac ccc ttt gac cag gac atc tac       1342
   Ala Phe Glu Leu Asp Thr Asp Gly Asn Pro Phe Asp Gln Asp Ile Tyr
                   360                 365                 370
   ggg cgc gag gag ctg cgc agc ccc aag ctg ttc tac gcc gac cac ccc      1390
   Gly Arg Glu Glu Leu Arg Ser Pro Lys Leu Phe Tyr Ala Asp His Pro
               375                 380                 385
   ttc atc ttc cta gtg cgg gac acc caa agc ggc tcc ctg cta ttc att      1438
   Phe Ile Phe Leu Val Arg Asp Thr Gln Ser Gly Ser Leu Leu Phe Ile
           390                 395                 400
   ggg cgc ctg gtc cgg cct aag ggt gac aag atg cga gac gag tta tag      1486
   Gly Arg Leu Val Arg Pro Lys Gly Asp Lys Met Arg Asp Glu Leu
       405                 410                 415
   ggcctcaggg tgcacacagg atggcaggag gcatccaaag gctcctgaga cacatgggtg   1546
   ctattggggt tgggggggag gtgaggtacc agccttggat actccatggg gtgggggtgg   1606
   aaaaacagac cggggttccc gtgtgcctga gcggaccttc ccagctagaa ttcactccac   1666
   ttggacatgg gccccagata ccatgatgct gagcccggaa actccacatc ctgtgggacc   1726
   tgggccatag tcattctgcc tgccctgaaa gtcccagatc aagcctgcct caatcagtat   1786
   tcatatttat agccaggtac cttctcacct gtgagaccaa attgagctag gggggtcagc   1846
   cagccctctt ctgacactaa aacacctcag ctgcctcccc agctctatcc caacctctcc   1906
   caactataaa actaggtgct gcagcccctg ggaccaggca cccccagaat gacctggccg   1966
   cagtgaggcg gattgagaag gagctcccag gaggggcttc tgggcagact ctggtcaaga   2026
   agcatcgtgt ctggcgttgt ggggatgaac tttttgtttt gtttcttcct tttttagttc   2086
   ttcaaagata gggagggaag ggggaacatg agcctttgtt gctatcaatc caagaactta   2146
   tttgtacatt ttttttttca ataaaacttt tccaatgaca ttttgttgga gcgtggaaaa   2206
   aa                                                                   2208
<210> 2
   <211> 418
   <212> PRT
   <213> Homo sapiens
<400> 2
   Met Arg Ser Leu Leu Leu Leu Ser Ala Phe Cys Leu Leu Glu Ala Ala
   1               5                   10                  15
   Leu Ala Ala Glu Val Lys Lys Pro Ala Ala Ala Ala Ala Pro Gly Thr
               20                  25                  30
   Ala Glu Lys Leu Ser Pro Lys Ala Ala Thr Leu Ala Glu Arg Ser Ala
           35                  40                  45
   Gly Leu Ala Phe Ser Leu Tyr Gln Ala Met Ala Lys Asp Gln Ala Val
       50                  55                  60
   Glu Asn Ile Leu Val Ser Pro Val Val Val Ala Ser Ser Leu Gly Leu
   65                  70                  75                  80
   Val Ser Leu Gly Gly Lys Ala Thr Thr Ala Ser Gln Ala Lys Ala Val
                   85                  90                  95
   Leu Ser Ala Glu Gln Leu Arg Asp Glu Glu Val His Ala Gly Leu Gly
               100                 105                 110
   Glu Leu Leu Arg Ser Leu Ser Asn Ser Thr Ala Arg Asn Val Thr Trp
           115                 120                 125
   Lys Leu Gly Ser Arg Leu Tyr Gly Pro Ser Ser Val Ser Phe Ala Asp
       130                 135                 140
   Asp Phe Val Arg Ser Ser Lys Gln His Tyr Asn Cys Glu His Ser Lys
   145                 150                 155                 160
   Ile Asn Phe Arg Asp Lys Arg Ser Ala Leu Gln Ser Ile Asn Glu Trp
                   165                 170                 175
   Ala Ala Gln Thr Thr Asp Gly Lys Leu Pro Glu Val Thr Lys Asp Val
               180                 185                 190
   Glu Arg Thr Asp Gly Ala Leu Leu Val Asn Ala Met Phe Phe Lys Pro
           195                 200                 205
   His Trp Asp Glu Lys Phe His His Lys Met Val Asp Asn Arg Gly Phe
       210                 215                 220
   Met Val Thr Arg Ser Tyr Thr Val Gly Val Met Met Met His Arg Thr
   225                 230                 235                 240
   Gly Leu Tyr Asn Tyr Tyr Asp Asp Glu Lys Glu Lys Leu Gln Ile Val
                   245                 250                 255
   Glu Met Pro Leu Ala His Lys Leu Ser Ser Leu Ile Ile Leu Met Pro
               260                 265                 270
   His His Val Glu Pro Leu Glu Arg Leu Glu Lys Leu Leu Thr Lys Glu
           275                 280                 285
   Gln Leu Lys Ile Trp Met Gly Lys Met Gln Lys Lys Ala Val Ala Ile
       290                 295                 300
   Ser Leu Pro Lys Gly Val Val Glu Val Thr His Asp Leu Gln Lys His
   305                 310                 315                 320
   Leu Ala Gly Leu Gly Leu Thr Glu Ala Ile Asp Lys Asn Lys Ala Asp
                   325                 330                 335
   Leu Ser Arg Met Ser Gly Lys Lys Asp Leu Tyr Leu Ala Ser Val Phe
               340                 345                 350
   His Ala Thr Ala Phe Glu Leu Asp Thr Asp Gly Asn Pro Phe Asp Gln
           355                 360                 365
   Asp Ile Tyr Gly Arg Glu Glu Leu Arg Ser Pro Lys Leu Phe Tyr Ala
       370                 375                 380
   Asp His Pro Phe Ile Phe Leu Val Arg Asp Thr Gln Ser Gly Ser Leu
   385                 390                 395                 400
   Leu Phe Ile Gly Arg Leu Val Arg Pro Lys Gly Asp Lys Met Arg Asp
                   405                 410                 415
   Glu Leu
<210> 3
   <211> 5927
   <212> DNA
   <213> Homo sapens
<220>
   <221> CDS
   <222> (127)..(4521)
   <223>
<400> 3
tcgtcggagc agacgggagt ttctcctcgg ggtcggagca ggaggcacgc ggagtgtgag     60
   gccacgcatg agcggacgct aaccccctcc ccagccacaa agagtctaca tgtctagggt    120
   ctagac atg ttc agc ttt gtg gac ctc cgg ctc ctg ctc ctc tta gcg      168
   gcc acc gcc ctc ctg acg cac ggc caa gag gaa ggc caa gtc gag ggc      216
   caa gac gaa gac atc cca cca atc acc tgc gta cag aac ggc ctc agg      264
   tac cat gac cga gac gtg tgg aaa ccc gag ccc tgc cgg atc tgc gtc      312
   tgc gac aac ggc aag gtg ttg tgc gat gac gtg atc tgt gac gag acc      360
   aag aac tgc ccc ggc gcc gaa gtc ccc gag ggc gag tgc tgt ccc gtc      408
   tgc ccc gac ggc tca gag tca ccc acc gac caa gaa acc acc ggc gtc      456
   gag gga ccc aag gga gac act ggc ccc cga ggc cca agg gga ccc gca      504
   ggc ccc cct ggc cga gat ggc atc cct gga cag cct gga ctt ccc gga      552
   ccc ccc gga ccc ccc gga cct ccc gga ccc cct ggc ctc gga gga aac      600
   ttt gct ccc cag ctg tct tat ggc tat gat gag aaa tca acc gga gga      648
   att tcc gtg cct ggc ccc atg ggt ccc tct ggt cct cgt ggt ctc cct      696
   ggc ccc cct ggt gca cct ggt ccc caa ggc ttc caa ggt ccc cct ggt      744
   gag cct ggc gag cct gga gct tca ggt ccc atg ggt ccc cga ggt ccc      792
   cca ggt ccc cct gga aag aat gga gat gat ggg gaa gct gga aaa cct      840
   ggt cgt cct ggt gag cgt ggg cct cct ggg cct cag ggt gct cga gga      888
   ttg ccc gga aca gct ggc ctc cct gga atg aag gga cac aga ggt ttc      936
   agt ggt ttg gat ggt gcc aag gga gat gct ggt cct gct ggt cct aag      984
   ggt gag cct ggc agc cct ggt gaa aat gga gct cct ggt cag atg ggc     1032
   ccc cgt ggc ctg cct ggt gag aga ggt cgc cct gga gcc cct ggc cct     1080
   gct ggt gct cgt gga aat gat ggt gct act ggt gct gcc ggg ccc cct      1128
   ggt ccc acc ggc ccc gct ggt cct cct ggc ttc cct ggt gct gtt ggt      1176
   gct aag ggt gaa gct ggt ccc caa ggg ccc cga ggc tct gaa ggt ccc      1224
   cag ggt gtg cgt ggt gag cct ggc ccc cct ggc cct gct ggt gct gct      1272
   ggc cct gct gga aac cct ggt gct gat gga cag cct ggt gct aaa ggt      1320
   gcc aat ggt gct cct ggt att gct ggt gct cct ggc ttc cct ggt gcc      1368
   cga ggc ccc tct gga ccc cag ggc ccc ggc ggc cct cct ggt ccc aag      1416
   ggt aac agc ggt gaa cct ggt gct cct ggc agc aaa gga gac act ggt      1464
   gct aag gga gag cct ggc cct gtt ggt gtt caa gga ccc cct ggc cct      1512
   gct gga gag gaa gga aag cga gga gct cga ggt gaa ccc gga ccc act      1560
   ggc ctg ccc gga ccc cct ggc gag cgt ggt gga cct ggt agc cgt ggt      1608
   ttc cct ggc gca gat ggt gtt gct ggt ccc aag ggt ccc gct ggt gaa      1656
   cgt ggt tct cct ggc cct gct ggc ccc aaa gga tct cct ggt gaa gct      1704
   ggt cgt ccc ggt gaa gct ggt ctg cct ggt gcc aag ggt ctg act gga      1752
   agc cct ggc agc cct ggt cct gat ggc aaa act ggc ccc cct ggt ccc      1800
   gcc ggt caa gat ggt cgc ccc gga ccc cca ggc cca cct ggt gcc cgt      1848
   ggt cag gct ggt gtg atg gga ttc cct gga cct aaa ggt gct gct gga      1896
   gag ccc ggc aag gct gga gag cga ggt gtt ccc gga ccc cct ggc gct      1944
   gtc ggt cct gct ggc aaa gat gga gag gct gga gct cag gga ccc cct      1992
   ggc cct gct ggt ccc gct ggc gag aga ggt gaa caa ggc cct gct ggc      2040
   tcc ccc gga ttc cag ggt ctc cct ggt cct gct ggt cct cca ggt gaa      2088
   gca ggc aaa cct ggt gaa cag ggt gtt cct gga gac ctt ggc gcc cct      2136
   ggc ccc tct gga gca aga ggc gag aga ggt ttc cct ggc gag cgt ggt      2184
   gtg caa ggt ccc cct ggt cct gct ggt ccc cga ggg gcc aac ggt gct      2232
   ccc ggc aac gat ggt gct aag ggt gat gct ggt gcc cct gga gct ccc      2280
   ggt agc cag ggc gcc cct ggc ctt cag gga atg cct ggt gaa cgt ggt      2328
   gca gct ggt ctt cca ggg cct aag ggt gac aga ggt gat gct ggt ccc      2376
   aaa ggt gct gat ggc tct cct ggc aaa gat ggc gtc cgt ggt ctg act      2424
   ggc ccc att ggt cct cct ggc cct gct ggt gcc cct ggt gac aag ggt      2472
   gaa agt ggt ccc agc ggc cct gct ggt ccc act gga gct cgt ggt gcc      2520
   ccc gga gac cgt ggt gag cct ggt ccc ccc ggc cct gct ggc ttt gct      2568
   ggc ccc cct ggt gct gac ggc caa cct ggt gct aaa ggc gaa cct ggt      2616
   gat gct ggt gct aaa ggc gat gct ggt ccc cct ggc cct gcc gga ccc      2664
   gct gga ccc cct ggc ccc att ggt aat gtt ggt gct cct gga gcc aaa      2712
   ggt gct cgc ggc agc gct ggt ccc cct ggt gct act ggt ttc cct ggt      2760
   gct gct ggc cga gtc ggt cct cct ggc ccc tct gga aat gct gga ccc      2808
   cct ggc cct cct ggt cct gct ggc aaa gaa ggc ggc aaa ggt ccc cgt      2856
   ggt gag act ggc cct gct gga cgt cct ggt gaa gtt ggt ccc cct ggt      2904
   ccc cct ggc cct gct ggc gag aaa gga tcc cct ggt gct gat ggt cct      2952
   gct ggt gct cct ggt act ccc ggg cct caa ggt att gct gga cag cgt      3000
   ggt gtg gtc ggc ctg cct ggt cag aga gga gag aga ggc ttc cct ggt      3048
   ctt cct ggc ccc tct ggt gaa cct ggc aaa caa ggt ccc tct gga gca      3096
   agt ggt gaa cgt ggt ccc cct ggt ccc atg  ggc ccc cct gga ttg  gct    3144
   gga ccc cct ggt  gaa tct gga cgt gag  ggg gct cct ggt gcc  gaa       3189
   ggt tcc cct gga  cga gac ggt tct cct  ggc gcc aag ggt gac  cgt       3234
   ggt gag acc ggc  ccc gct gga ccc cct  ggt gct cct ggt gct  cct       3279
   ggt gcc cct ggc  ccc gtt ggc cct gct  ggc aag agt ggt gat  cgt       3324
   ggt gag act ggt  cct gct ggt ccc gcc  ggt cct gtc ggc cct  gtt       3369
   ggc gcc cgt ggc  ccc gcc gga ccc caa  ggc ccc cgt ggt gac  aag        3414
   ggt gag aca ggc  gaa cag ggc gac aga  ggc ata aag ggt cac  cgt        3459
   ggc ttc tct ggc  ctc cag ggt ccc cct  ggc cct cct ggc tct  cct        3504
   ggt gaa caa ggt  ccc tct gga gcc tct  ggt cct gct ggt ccc  cga        3549
   ggt ccc cct ggc  tct gct ggt gct cct  ggc aaa gat gga ctc  aac        3594
   ggt ctc cct ggc  ccc att ggg ccc cct  ggt cct cgc ggt cgc  act        3639
   ggt gat gct ggt  cct gtt ggt ccc ccc  ggc cct cct gga cct  cct        3684
   ggt ccc cct ggt  cct ccc agc gct ggt  ttc gac ttc agc ttc  ctg        3729
   ccc cag cca cct  caa gag aag gct cac  gat ggt ggc cgc tac  tac        3774
   cgg gct gat gat  gcc aat gtg gtt cgt  gac cgt gac ctc gag  gtg        3819
   gac acc acc ctc  aag agc ctg agc cag  cag atc gag aac atc  cgg        3864
   agc cca gag ggc  agc cgc aag aac ccc  gcc cgc acc tgc cgt  gac        3909
   ctc aag atg tgc  cac tct gac tgg aag  agt gga gag tac tgg  att        3954
   gac ccc aac caa  ggc tgc aac ctg gat  gcc atc aaa gtc ttc  tgc        3999
   aac atg gag act  ggt gag acc tgc gtg  tac ccc act cag ccc  agt        4044
   gtg gcc cag aag  aac tgg tac atc agc  aag aac ccc aag gac  aag      4089
   agg cat gtc tgg  ttc ggc gag agc atg  acc gat gga ttc cag  ttc      4134
   gag tat ggc ggc  cag ggc tcc gac cct  gcc gat gtg gcc atc  cag      4179
   ctg acc ttc ctg  cgc ctg atg tcc acc  gag gcc tcc cag aac  atc      4224
   acc tac cac tgc  aag aac agc gtg gcc  tac atg gac cag cag  act      4269
   ggc aac ctc aag  aag gcc ctg ctc ctc  cag ggc tcc aac gag  atc      4314
   gag atc cgc gcc  gag ggc aac agc cgc  ttc acc tac agc gtc  act      4359
   gtc gat ggc tgc  acg agt cac acc gga  gcc tgg ggc aag aca  gtg      4404
   att gaa tac aaa  acc acc aag acc tcc  cgc ctg ccc atc atc  gat      4449
   gtg gcc ccc ttg  gac gtt ggt gcc cca  gac cag gaa ttc ggc  ttc      4494
   gac gtt ggc cct  gtc tgc ttc ctg taa actccctcca tcccaacctg          4541
   gctccctccc acccaaccaa ctttcccccc aacccggaaa cagacaagca acccaaactg   4601
   aaccccctca aaagccaaaa aatgggagac aatttcacat ggactttgga aaatattttt   4661
   ttcctttgca ttcatctctc aaacttagtt tttatctttg accaaccgaa catgaccaaa   4721
   aaccaaaagt gcattcaacc ttaccaaaaa aaaaaaaaaa aaaagaataa ataaataact   4781
   ttttaaaaaa ggaagcttgg tccacttgct tgaagaccca tgcgggggta agtccctttc   4841
   tgcccgttgg gcttatgaaa ccccaatgct gccctttctg ctcctttctc cacacccccc   4901
   ttggggcctc ccctccactc cttcccaaat ctgtctcccc agaagacaca ggaaacaatg   4961
   tattgtctgc ccagcaatca aaggcaatgc tcaaacaccc aagtggcccc caccctcagc   5021
   ccgctcctgc ccgcccagca cccccaggcc ctgggggacc tggggttctc agactgccaa   5081
   agaagccttg ccatctggcg ctcccatggc tcttgcaaca tctccccttc gtttttgagg   5141
   gggtcatgcc gggggagcca ccagcccctc actgggttcg gaggagagtc aggaagggcc 5201
   acgacaaagc agaaacatcg gatttgggga acgcgtgtca atcccttgtg ccgcagggct 5261
   gggcgggaga gactgttctg ttccttgtgt aactgtgttg ctgaaagact acctcgttct 5321
   tgtcttgatg tgtcaccggg gcaactgcct gggggcgggg atgggggcag ggtggaagcg 5381
   gctccccatt ttataccaaa ggtgctacat ctatgtgatg ggtggggtgg ggagggaatc 5441
   actggtgcta tagaaattga gatgcccccc caggccagca aatgttcctt tttgttcaaa 5501
   gtctattttt attccttgat atttttcttt tttttttttt ttttttgtgg atggggactt 5561
   gtgaattttt ctaaaggtgc tatttaacat gggaggagag cgtgtgcggc tccagcccag 5621
   cccgctgctc actttccacc ctctctccac ctgcctctgg cttctcaggc ctctgctctc 5681
   cgacctctct cctctgaaac cctcctccac agctgcagcc catcctcccg gctccctcct 5741
   agtctgtcct gcgtcctctg tccccgggtt tcagagacaa cttcccaaag cacaaagcag 5801
   tttttccccc taggggtggg aggaagcaaa agactctgta cctattttgt atgtgtataa 5861
   taatttgaga tgtttttaat tattttgatt gctggaataa agcatgtgga aatgacccaa 5921
   acataa 5927
<210> 4
   <211> 1464
   <212> PRT
   <213> Homo sapens
<400> 4
<210> 5
   <211> 5411
   <212> DNA
   <213> Homo sapiens
<220>
   <221> CDS
   <222> (472)..(4572)
   <223>
<400> 5
gtgtcccata gtgtttccaa acttggaaag ggcgggggag ggcgggagga tgcggagggc      60
   ggaggtatgc agacaacgag tcagagtttc cccttgaaag cctcaaaagt gtccacgtcc     120
   tcaaaaagaa tggaaccaat ttaagaagcc agccccgtgg ccacgtccct tcccccattc     180
   gctccctcct ctgcgccccc gcaggctcct cccagctgtg gctgcccggg cccccagccc     240
   cagccctccc attggtggag gcccttttgg aggcacccta gggccaggga aacttttgcc     300
   gtataaatag ggcagatccg ggctttatta ttttagcacc acggcagcag gaggtttcgg     360
   ctaagttgga ggtactggcc acgactgcat gcccgcgccc gccaggtgat acctccgccg     420
   gtgacccagg ggctctgcga cacaaggagt ctgcatgtct aagtgctaga c atg ctc     477
   agc ttt gtg gat acg cgg act ttg ttg ctg ctt gca gta acc tta tgc      525
   cta gca aca tgc caa tct tta caa gag gaa act gta aga aag ggc cca      573
   gcc gga gat aga gga cca cgt gga gaa agg ggt cca cca ggc ccc cca      621
   ggc aga gat ggt gaa gat ggt ccc aca ggc cct cct ggt cca cct ggt      669
   cct cct ggc ccc cct ggt ctc ggt ggg aac ttt gct gct cag tat gat      717
   gga aaa gga gtt gga ctt ggc cct gga cca atg ggc tta atg gga cct      765
   aga ggc cca cct ggt gca gct gga gcc cca ggc cct caa ggt ttc caa      813
   gga cct gct ggt gag cct ggt gaa cct ggt caa act ggt cct gca ggt      861
   gct cgt ggt cca gct ggc cct cct ggc aag gct ggt gaa gat ggt cac      909
   cct gga aaa ccc gga cga cct ggt gag aga gga gtt gtt gga cca cag      957
   ggt gct cgt ggt ttc cct gga act cct gga ctt cct ggc ttc aaa ggc     1005
   att agg gga cac aat ggt ctg gat gga ttg aag gga cag ccc ggt gct     1053
   cct ggt gtg aag ggt gaa cct ggt gcc cct ggt gaa aat gga act cca     1101
   ggt caa aca gga gcc cgt ggg ctt cct ggt gag aga gga cgt gtt ggt     1149
   gcc cct ggc cca gct ggt gcc cgt ggc agt gat gga agt gtg ggt ccc     1197
   gtg ggt cct gct ggt ccc att ggg tct gct ggc cct cca ggc ttc cca     1245
   ggt gcc cct ggc ccc aag ggt gaa att gga gct gtt ggt aac gct ggt      1293
   cct gct ggt ccc gcc ggt ccc cgt ggt gaa gtg ggt ctt cca ggc ctc      1341
   tcc ggc ccc gtt gga cct cct ggt aat cct gga gca aac ggc ctt act      1389
   ggt gcc aag ggt gct gct ggc ctt ccc ggc gtt gct ggg gct ccc ggc      1437
   ctc cct gga ccc cgc ggt att cct ggc cct gtt ggt gct gcc ggt gct      1485
   act ggt gcc aga gga ctt gtt ggt gag cct ggt cca gct ggc tcc aaa      1533
   gga gag agc ggt aac aag ggt gag ccc ggc tct gct ggg ccc caa ggt      1581
   cct cct ggt ccc agt ggt gaa gaa gga aag aga ggc cct aat ggg gaa      1629
   gct gga tct gcc ggc cct cca gga cct cct ggg ctg aga ggt agt cct      1677
   ggt tct cgt ggt ctt cct gga gct gat ggc aga gct ggc gtc atg ggc      1725
   cct cct ggt agt cgt ggt gca agt ggc cct gct gga gtc cga gga cct      1773
   aat gga gat gct ggt cgc cct ggg gag cct ggt ctc atg gga ccc aga      1821
   ggt ctt cct ggt tcc cct gga aat atc ggc ccc gct gga aaa gaa ggt      1869
   cct gtc ggc ctc cct ggc atc gac ggc agg cct ggc cca att ggc cca      1917
   gct gga gca aga gga gag cct ggc aac att gga ttc cct gga ccc aaa      1965
   ggc ccc act ggt gat cct ggc aaa aac ggt gat aaa ggt cat gct ggt      2013
   ctt gct ggt gct cgg ggt gct cca ggt cct gat gga aac aat ggt gct      2061
   cag gga cct cct gga cca cag ggt gtt caa ggt gga aaa ggt gaa cag      2109
   ggt ccc cct ggt cct cca ggc ttc cag ggt ctg cct ggc ccc tca ggt      2157
   ccc gct ggt gaa gtt ggc aaa cca gga gaa agg ggt ctc cat ggt gag      2205
   ttt ggt ctc cct ggt cct gct ggt cca aga ggg gaa cgc ggt ccc cca      2253
   ggt gag agt ggt gct gcc ggt cct act ggt cct att gga agc cga ggt      2301
   cct tct gga ccc cca ggg cct gat gga aac aag ggt gaa cct ggt gtg      2349
   gtt ggt gct gtg ggc act gct ggt cca tct ggt cct agt gga ctc cca      2397
   gga gag agg ggt gct gct ggc ata cct gga ggc aag gga gaa aag ggt      2445
   gaa cct ggt ctc aga ggt gaa att ggt aac cct ggc aga gat ggt gct      2493
   cgt ggt gct cct ggt gct gta ggt gcc cct ggt cct gct gga gcc aca      2541
   ggt gac cgg ggc gaa gct ggg gct gct ggt cct gct ggt cct gct ggt      2589
   cct cgg gga agc cct ggt gaa cgt ggt gag gtc ggt cct gct ggc ccc      2637
   aat gga ttt gct ggt cct gct ggt gct gct ggt caa cct ggt gct aaa      2685
   gga gaa aga gga gcc aaa ggg cct aag ggt gaa aac ggt gtt gtt ggt      2733
   ccc aca ggc ccc gtt gga gct gct ggc cca gct ggt cca aat ggt ccc 2781
   ccc ggt cct gct gga agt cgt ggt gat gga ggc ccc cct ggt atg act 2829
   ggt ttc cct ggt gct gct gga cgg act ggt ccc cca gga ccc tct ggt 2877
   att tct ggc cct cct ggt ccc cct ggt cct gct ggg aaa gaa ggg ctt 2925
   cgt ggt cct cgt ggt gac caa ggt cca gtt ggc cga act gga gaa gta 2973
   ggt gca gtt ggt ccc cct ggc ttc gct ggt gag aag ggt ccc tct gga 3021
   gag gct ggt act gct gga cct cct ggc act cca ggt cct cag ggt ctt 3069
   ctt ggt gct cct ggt att ctg ggt ctc cct ggc tcg aga ggt gaa cgt 3117
   ggt cta cca ggt gtt gct ggt gct gtg ggt gaa cct ggt cct ctt ggc 3165
   att gcc ggc cct cct ggg gcc cgt ggt cct cct ggt gct gtg ggt agt 3213
   cct gga gtc aac ggt gct cct ggt gaa gct ggt cgt gat ggc aac cct 3261
   ggg aac gat ggt ccc cca ggt cgc gat ggt caa ccc gga cac aag gga 3309
   gag cgc ggt tac cct ggc aat att ggt ccc gtt ggt gct gca ggt gca 3357
   cct ggt cct cat ggc ccc gtg ggt cct gct ggc aaa cat gga aac cgt 3405
   ggt gaa act ggt cct tct ggt cct gtt ggt cct gct ggt gct gtt ggc 3453
   cca aga ggt cct agt ggc cca caa ggc att cgt ggc gat aag gga 3498
   gag  ccc ggt gaa aag ggg  ccc aga ggt ctt cct  ggc tta aag gga      3543
   cac  aat gga ttg caa ggt  ctg cct ggt atc gct  ggt cac cat ggt      3588
   gat  caa ggt gct cct ggc  tcc gtg ggt cct gct  ggt cct agg ggc      3633
   cct  gct ggt cct tct ggc  cct gct gga aaa gat  ggt cgc act gga      3678
   cat  cct ggt aca gtt gga  cct gct ggc att cga  ggc cct cag ggt      3723
   cac  caa ggc cct gct ggc  ccc cct ggt ccc cct  ggc cct cct gga      3768
   cct  cca ggt gta agc ggt  ggt ggt tat gac ttt  ggt tac gat gga      3813
   gac  ttc tac agg gct gac  cag cct cgc tca gca  cct tct ctc aga      3858
   ccc  aag gac tat gaa gtt  gat gct act ctg aag  tct ctc aac aac      3903
   cag  att gag acc ctt ctt  act cct gaa ggc tct  aga aag aac cca      3948
   gct  cgc aca tgc cgt gac  ttg aga ctc agc cac  cca gag tgg agc      3993
   agt  ggt tac tac tgg att  gac cct aac caa gga  tgc act atg gat      4038
   gct  atc aaa gta tac tgt  gat ttc tct act ggc  gaa acc tgt atc      4083
   cgg  gcc caa cct gaa aac  atc cca gcc aag aac  tgg tat agg agc      4128
   tcc  aag gac aag aaa cac  gtc tgg cta gga gaa  act atc aat gct      4173
   ggc  agc cag ttt gaa tat  aat gta gaa gga gtg  act tcc aag gaa      4218
   atg gct acc caa ctt gcc ttc atg cgc ctg ctg gcc aac tat gcc      4263
   tct cag aac atc acc tac cac tgc aag aac agc att gca tac atg      4308
   gat gag gag act ggc aac ctg aaa aag gct gtc att cta cag ggc      4353
   tct aat gat gtt gaa ctt gtt gct gag ggc aac agc agg ttc act      4398
   tac act gtt ctt gta gat ggc tgc tct aaa aag aca aat gaa tgg      4443
   gga aag aca atc att gaa tac aaa aca aat aag cca tca cgc ctg      4488
   ccc ttc ctt gat att gca cct ttg gac atc ggt ggt gct gac cag      4533
   gaa ttc ttt gtg gac att ggc cca gtc tgt ttc aaa taa atgaactcaa   4582
   tctaaattaa aaaagaaaga aatttgaaaa aactttctct ttgccatttc ttcttcttct   4642
   tttttaactg aaagctgaat ccttccattt cttctgcaca tctacttgct taaattgtgg   4702
   gcaaaagaga aaaagaagga ttgatcagag cattgtgcaa tacagtttca ttaactcctt   4762
   cccccgctcc cccaaaaatt tgaatttttt tttcaacact cttacacctg ttatggaaaa   4822
   tgtcaacctt tgtaagaaaa ccaaaataaa aattgaaaaa taaaaaccat aaacatttgc   4882
   accacttgtg gcttttgaat atcttccaca gagggaagtt taaaacccaa acttccaaag   4942
   gtttaaacta cctcaaaaca ctttcccatg agtgtgatcc acattgttag gtgctgacct   5002
   agacagagat gaactgaggt ccttgttttg ttttgttcat aatacaaagg tgctaattaa   5062
   tagtatttca gatacttgaa gaatgttgat ggtgctagaa gaatttgaga agaaatactc   5122
   ctgtattgag ttgtatcgtg tggtgtattt tttaaaaaat ttgatttagc attcatattt   5182
   tccatcttat tcccaattaa aagtatgcag attatttgcc caaatcttct tcagattcag   5242
   catttgttct ttgccagtct cattttcatc ttcttccatg gttccacaga agctttgttt   5302
   cttgggcaag cagaaaaatt aaattgtacc tattttgtat atgtgagatg tttaaataaa   5362
   ttgtgaaaaa aatgaaataa agcatgtttg gttttccaaa agaacatat               5411
<210> 6
   <211> 1366
   <212> PRT
   <213> Homo sapiens
<400> 6